# EUROPEAN PATENT SPECIFICATION

(11) **EP 2 120 859 B1**
(45) Date of publication and mention of the grant of the patent: **20.11.2013**
(21) Application number: 07863119.9
(22) Date of filing: 19.12.2007
(51) Int. Cl.: A61K 9/00, A61K 9/14, A61K 38/00, A61K 38/18

(54) **SUSTAINED-RELEASE FORMULATIONS COMPRISING BMP-7 CRYSTALS**
FORMULIERUNGEN MIT VERZÖGERTER FREISETZUNG MIT KRISTALLEN VON BMP-7
FORMULATIONS À LIBÉRATION ENTRETENUE COMPRENANT DES CRISTAUX DE BMP-7

(30) Priority: 21.12.2006 US 876292 P
(43) Date of publication of application: 25.11.2009
(73) Proprietor: STRYKER CORPORATION, Kalamazoo Michigan 49005 (US)
(72) Inventor: JAWOROWICZ, Warren, Bolton, MA 01740 (US)
(74) Representative: Lock, Graham James
(86) International application number: PCT/US2007/025956
(87) International publication number: WO 2008/082563

(56) References cited:
- WO-A-03/006049
- WO-A-2004/030650
- WO-A-2004/060920
- US-A- 5 595 760
- US-A1- 2006 257 492
- US-B1- 6 503 534
- JEN A ET AL: "Transforming growth factor beta-3 crystals as reservoirs for slow release of active TGF-beta3" JOURNAL OF CONTROLLED RELEASE, ELSEVIER, AMSTERDAM, NL, vol. 78, no. 1-3, 17 January 2002 (2002-01-17), pages 25-34, XP004329803 ISSN: 0168-3659
- LUGINBUEHL V ET AL: "Localized delivery of growth factors for bone repair" EUROPEAN JOURNAL OF PHARMACEUTICS AND BIOPHARMACEUTICS, ELSEVIER SCIENCE PUBLISHERS B.V., AMSTERDAM, NL, vol. 58, no. 2, 1 September 2004 (2004-09-01), pages 197-208, XP004526306 ISSN: 0939-6411
- BEALS ET AL: "Enhancing exposure of protein therapeutics" DRUG DISCOVERY TODAY: TECHNOLOGIES, ELSEVIER, vol. 3, no. 1, 1 April 2006 (2006-04-01), pages 87-94, XP005409090 ISSN: 1740-6749
- JELIC M ET AL: "Regeneration of articular cartilage chondral defects by osteogenic protein-1 (bone morphogenetic protein-7) in sheep" GROWTH FACTORS, HARWOOD ACADEMIC PUBLISHERS GMBH, XX, vol. 19, no. 2, 1 January 2001 (2001-01-01), pages 101-113, XP008093561 ISSN: 0897-7194
- BASU S K ET AL: "PROTEIN CRYSTALS FOR THE DELIVERY OF BIOPHARMACEUTICALS" EXPERT OPINION ON BIOLOGICAL THERAPY, ASHLEY, LONDON, GB, vol. 4, no. 3, 1 March 2004 (2004-03-01), pages 301-317, XP009040451 ISSN: 1471-2598
- PECHENOV S ET AL: "Injectable controlled release formulations incorporating protein crystals" JOURNAL OF CONTROLLED RELEASE, ELSEVIER, AMSTERDAM, NL, vol. 96, no. 1, 16 April 2004 (2004-04-16), pages 149-158, XP004500740 ISSN: 0168-3659
- CENDROS ET AL: "Pharmacokinetics and population pharmacodynamic analysis of lanreotide Autogel" METABOLISM, CLINICAL AND EXPERIMENTAL, W.B. SAUNDERS CO., PHILADELPHIA, PA, US, vol. 54, no. 10, 1 October 2005 (2005-10-01), pages 1276-1281, XP005067925 ISSN: 0026-0495
- PHILLIPS ET AL: "In vivo BMP-7 (OP-1) enhancement of osteoporotic vertebral bodies in an ovine model" THE SPINE JOURNAL, ELSEVIER, vol. 6, no. 5, 1 September 2006 (2006-09-01), pages 500-506, XP005612783 ISSN: 1529-9430
- GRIFFITH D L ET AL: "THREE-DIMENSIONAL STRUCTURE OF RECOMBINANT HUMAN OSTEOGENIC PROTEIN 1: STRUCTURAL PARADIGM FOR THE TRANSFORMING GROWTH FACTOR BETA SUPERFAMILY", PROCEEDINGS OF THE NATIONAL ACADEMY OF SCIENCES, NATIONAL ACADEMY OF SCIENCES, US, vol. 93, no. 2, 23 January 1996 (1996-01-23), pages 878-838, XP002035127, ISSN: 0027-8424, DOI: 10.1073/PNAS.93.2.878
- CELESTE A J ET AL: "IDENTIFICATION OF TRANSFORMING GROWTH FACTOR B FAMILY MEMBERS PRESENT IN BONE-INDUCTIVE PROTEIN PURIFIED FROM BOVINE BONE", PROCEEDINGS OF THE NATIONAL ACADEMY OF SCIENCES, NATIONAL ACADEMY OF SCIENCES, US, vol. 87, no. 24, 1 December 1990 (1990-12-01), pages 9843-9847, XP000168624, ISSN: 0027-8424, DOI: 10.1073/PNAS.87.24.9843
- FIDDIS ET AL.: ANN RHEUM DIS, vol. 42, 1983, pages S12-S15,

### SEQUENCE LISTING

<110> Stryker Corporation Jaworowicz, Warren
<120> sustained-Release Formulations Comprising Crystals, Macromolecular Gels, and Particulate suspensions of Biologic Agents
<130> STK-088PC
<150> 60/876,292
   <151> 2006-12-21
<160> 18
<170> PatentIn version 3.3
<210> 1
   <211> 396
   <212> PRT
   <213> Homo sapiens
<400> 1
   Met Val Ala Gly Thr Arg Cys Leu Leu Ala Leu Leu Leu Pro Gln Val
   1 5 10 15
   Leu Leu Gly Gly Ala Ala Gly Leu Val Pro Glu Leu Gly Arg Arg Lys
   20 25 30
   Phe Ala Ala Ala Ser Ser Gly Arg Pro Ser Ser Gln Pro Ser Asp Glu
   35 40 45
   Val Leu Ser Glu Phe Glu Leu Arg Leu Leu Ser Met Phe Gly Leu Lys
   50 55 60
   Gln Arg Pro Thr Pro Ser Arg Asp Ala Val Val Pro Pro Tyr Met Leu
   65 70 75 80
   Asp Leu Tyr Arg Arg His Ser Gly Gln Pro Gly Ser Pro Ala Pro Asp
   85 90 95
   His Arg Leu Glu Arg Ala Ala Ser Arg Ala Asn Thr Val Arg Ser Phe
   100 105 110
   His His Glu Glu Ser Leu Glu Glu Leu Pro Glu Thr Ser Gly Lys Thr
   115 120 125
   Thr Arg Arg Phe Phe Phe Asn Leu Ser Ser Ile Pro Thr Glu Glu Phe
   130 135 140
   Ile Thr Ser Ala Glu Leu Gln Val Phe Arg Glu Gln Met Gln Asp Ala
   145 150 155 160
   Leu Gly Asn Asn Ser Ser Phe His His Arg Ile Asn Ile Tyr Glu Ile
   165 170 175
   Ile Lys Pro Ala Thr Ala Asn Ser Lys Phe Pro Val Thr Arg Leu Leu
   180 185 190
   Asp Thr Arg Leu Val Asn Gln Asn Ala Ser Arg Trp Glu Ser Phe Asp
   195 200 205
   Val Thr Pro Ala Val Met Arg Trp Thr Ala Gln Gly His Ala Asn His
   210 215 220
   Gly Phe Val Val Glu Val Ala His Leu Glu Glu Lys Gln Gly Val Ser
   225 230 235 240
   Lys Arg His Val Arg Ile Ser Arg Ser Leu His Gln Asp Glu His Ser
   245 250 255
   Trp Ser Gln Ile Arg Pro Leu Leu Val Thr Phe Gly His Asp Gly Lys
   260 265 270
   Gly His Pro Leu His Lys Arg Glu Lys Arg Gln Ala Lys His Lys Gln
   275 280 285
   Arg Lys Arg Leu Lys Ser Ser Cys Lys Arg His Pro Leu Tyr Val Asp
   290 295 300
   Phe Ser Asp Val Gly Trp Asn Asp Trp Ile Val Ala Pro Pro Gly Tyr
   305 310 315 320
   His Ala Phe Tyr Cys His Gly Glu Cys Pro Phe Pro Leu Ala Asp His
   325 330 335
   Leu Asn Ser Thr Asn His Ala Ile Val Gln Thr Leu Val Asn Ser Val
   340 345 350
   Asn Ser Lys Ile Pro Lys Ala Cys Cys Val Pro Thr Glu Leu Ser Ala
   355 360 365
   Ile Ser Met Leu Tyr Leu Asp Glu Asn Glu Lys Val Val Leu Lys Asn
   370 375 380
   Tyr Gln Asp Met Val Val Glu Gly Cys Gly Cys Arg
   385 390 395
<210> 2
   <211> 3150
   <212> DNA
   <213> Homo sapiens
<400> 2
   ccacaaaggg cacttggccc cagggctagg agagcgaggg gagagcacag ccacccgcct 60
   cggcggcccg ggactcggct cgactcgccg gagaatgcgc ccgaggacga cggggcgcca 120
   gagccgcggt gctttcaact ggcgagcgcg aatgggggtg cactggagta aggcagagtg 180
   atgcgggggg gcaactcgcc tggcaccgag atcgccgccg tgcccttccc tggacccggc 240
   gtcgcccagg atggctgccc cgagccatgg gccgcggcgg agctagcgcg gagcgcccga 300
   ccctcgaccc ccgagtcccg gagccggccc cgcgcggggc cacgcgtccc tcgggcgctg 360
   gttcctaagg aggacgacag caccagcttc tcctttctcc cttcccttcc ctgccccgca 420
   ctcctccccc tgctcgctgt tgttgtgtgt cagcacttgg ctggggactt cttgaacttg 480
   cagggagaat aacttgcgca ccccactttg cgccggtgcc tttgccccag cggagcctgc 540
   ttcgccatct ccgagcccca ccgcccctcc actcctcggc cttgcccgac actgagacgc 600
   tgttcccagc gtgaaaagag agactgcgcg gccggcaccc gggagaagga ggaggcaaag 660
   aaaaggaacg gacattcggt ccttgcgcca ggtcctttga ccagagtttt tccatgtgga 720
   cgctctttca atggacgtgt ccccgcgtgc ttcttagacg gactgcggtc tcctaaaggt 780
   cgaccatggt ggccgggacc cgctgtcttc tagcgttgct gcttccccag gtcctcctgg 840
   gcggcgcggc tggcctcgtt ccggagctgg gccgcaggaa gttcgcggcg gcgtcgtcgg 900
   gccgcccctc atcccagccc tctgacgagg tcctgagcga gttcgagttg cggctgctca 960
   gcatgttcgg cctgaaacag agacccaccc ccagcaggga cgccgtggtg cccccctaca 1020
   tgctagacct gtatcgcagg cactcaggtc agccgggctc acccgcccca gaccaccggt 1080
   tggagagggc agccagccga gccaacactg tgcgcagctt ccaccatgaa gaatctttgg 1140
   aagaactacc agaaacgagt gggaaaacaa cccggagatt cttctttaat ttaagttcta 1200
   tccccacgga ggagtttatc acctcagcag agcttcaggt tttccgagaa cagatgcaag 1260
   atgctttagg aaacaatagc agtttccatc accgaattaa tatttatgaa atcataaaac 1320
   ctgcaacagc caactcgaaa ttccccgtga ccagactttt ggacaccagg ttggtgaatc 1380
   agaatgcaag caggtgggaa agttttgatg tcacccccgc tgtgatgcgg tggactgcac 1440
   agggacacgc caaccatgga ttcgtggtgg aagtggccca cttggaggag aaacaaggtg 1500
   tctccaagag acatgttagg ataagcaggt ctttgcacca agatgaacac agctggtcac 1560
   agataaggcc attgctagta acttttggcc atgatggaaa agggcatcct ctccacaaaa 1620
   gagaaaaacg tcaagccaaa cacaaacagc ggaaacgcct taagtccagc tgtaagagac 1680
   accctttgta cgtggacttc agtgacgtgg ggtggaatga ctggattgtg gctcccccgg 1740
   ggtatcacgc cttttactgc cacggagaat gcccttttcc tctggctgat catctgaact 1800
   ccactaatca tgccattgtt cagacgttgg tcaactctgt taactctaag attcctaagg 1860
   catgctgtgt cccgacagaa ctcagtgcta tctcgatgct gtaccttgac gagaatgaaa 1920
   aggttgtatt aaagaactat caggacatgg ttgtggaggg ttgtgggtgt cgctagtaca 1980
   gcaaaattaa atacataaat atatatatat atatatattt tagaaaaaag aaaaaaacaa 2040
   acaaacaaaa aaaccccacc ccagttgaca ctttaatatt tcccaatgaa gactttattt 2100
   atggaatgga atggaaaaaa aaacagctat tttgaaaata tatttatatc tacgaaaaga 2160
   agttgggaaa acaaatattt taatcagaga attattcctt aaagatttaa aatgtattta 2220
   gttgtacatt ttatatgggt tcaaccccag cacatgaagt ataatggtca gatttatttt 2280
   gtatttattt actattataa ccacttttta ggaaaaaaat agctaatttg tatttatatg   2340
   taatcaaaag aagtatcggg tttgtacata attttccaaa aattgtagtt gttttcagtt   2400
   gtgtgtattt aagatgaaaa gtctacatgg aaggttactc tggcaaagtg cttagcacgt   2460
   ttgctttttt gcagtgctac tgttgagttc acaagttcaa gtccagaaaa aaaaagtgga   2520
   taatccactc tgctgacttt caagattatt atattattca attctcagga atgttgcaga   2580
   gtgattgtcc aatccatgag aatttacatc cttattaggt ggaatatttg gataagaacc   2640
   agacattgct gatctattat agaaactctc ctcctgcccc ttaatttaca gaaagaataa   2700
   agcaggatcc atagaaataa ttaggaaaac gatgaacctg caggaaagtg aatgatggtt   2760
   tgttgttctt ctttcctaaa ttagtgatcc cttcaaaggg gctgatctgg ccaaagtatt   2820
   caataaaacg taagatttct tcattattga tattgtggtc atatatattt aaaattgata   2880
   tctcgtggcc ctcatcaagg gttggaaatt tatttgtgtt ttacctttac ctcatctgag   2940
   agctctttat tctccaaaga acccagtttt ctaacttttt gcccaacacg cagcaaaatt   3000
   atgcacatcg tgttttctgc ccaccctctg ttctctgacc tatcagcttg cttttctttc   3060
   caaggttgtg tgtttgaaca catttctcca aatgttaaac ctatttcaga taataaatat   3120
   caaatctctg gcatttcatt ctataaagtc                                    3150
<210> 3
   <211> 408
   <212> PRT
   <213> Homo sapiens
<400> 3
<210> 4
   <211> 1957
   <212> DNA
   <213> Homo sapiens
<400> 4
   aagaggagga aggaagatgc gagaaggcag aggaggaggg agggagggaa ggagcgcgga 60
   gcccggcccg gaagctaggt gagtgtggca tccgagctga gggacgcgag cctgagacgc 120
   cgctgctgct ccggctgagt atctagcttg tctccccgat gggattcccg tccaagctat 180
   ctcgagcctg cagcgccaca gtccccggcc ctcgcccagg ttcactgcaa ccgttcagag 240
   gtccccagga gctgctgctg gcgagcccgc tactgcaggg acctatggag ccattccgta 300
   gtgccatccc gagcaacgca ctgctgcagc ttccctgagc ctttccagca agtttgttca 360
   agattggctg tcaagaatca tggactgtta ttatatgcct tgttttctgt caagacacca 420
   tgattcctgg taaccgaatg ctgatggtcg ttttattatg ccaagtcctg ctaggaggcg 480
   cgagccatgc tagtttgata cctgagacgg ggaagaaaaa agtcgccgag attcagggcc 540
   acgcgggagg acgccgctca gggcagagcc atgagctcct gcgggacttc gaggcgacac 600
   ttctgcagat gtttgggctg cgccgccgcc cgcagcctag caagagtgcc gtcattccgg 660
   actacatgcg ggatctttac cggcttcagt ctggggagga ggaggaagag cagatccaca 720
   gcactggtct tgagtatcct gagcgcccgg ccagccgggc caacaccgtg aggagcttcc 780
   accacgaaga acatctggag aacatcccag ggaccagtga aaactctgct tttcgtttcc 840
   tctttaacct cagcagcatc cctgagaacg aggtgatctc ctctgcagag cttcggctct 900
   tccgggagca ggtggaccag ggccctgatt gggaaagggg cttccaccgt ataaacattt 960
   atgaggttat gaagccccca gcagaagtgg tgcctgggca cctcatcaca cgactactgg 1020
   acacgagact ggtccaccac aatgtgacac ggtgggaaac ttttgatgtg agccctgcgg 1080
   tccttcgctg gacccgggag aagcagccaa actatgggct agccattgag gtgactcacc 1140
   tccatcagac tcggacccac cagggccagc atgtcaggat tagccgatcg ttacctcaag 1200
   ggagtgggaa ttgggcccag ctccggcccc tcctggtcac ctttggccat gatggccggg 1260
   gccatgcctt gacccgacgc cggagggcca agcgtagccc taagcatcac tcacagcggg 1320
   ccaggaagaa gaataagaac tgccggcgcc actcgctcta tgtggacttc agcgatgtgg 1380
   gctggaatga ctggattgtg gccccaccag gctaccaggc cttctactgc catggggact 1440
   gcccctttcc actggctgac cacctcaact caaccaacca tgccattgtg cagaccctgg 1500
   tcaattctgt caattccagt atccccaaag cctgttgtgt gcccactgaa ctgagtgcca 1560
   tctccatgct gtacctggat gagtatgata aggtggtact gaaaaattat caggagatgg 1620
   tagtagaggg atgtgggtgc cgctgagatc aggcagtcct tgaggataga cagatataca 1680
   caccacacac acacaccaca tacaccacac acacacgttc ccatccactc acccacacac 1740
   tacacagact gcttccttat agctggactt ttatttaaaa aaaaaaaaaa aaaaggaaaa 1800
   aatccctaaa cattcacctt gaccttattt atgactttac gtgcaaatgt tttgaccata 1860
   ttgatcatat attttgacaa aatatattta taactacgta ttaaaagaaa aaaataaaat 1920
   gagtcattat tttaaaggta aaaaaaaaaa aaaaaaa 1957
<210> 5
   <211> 1748
   <212> DNA
   <213> Homo sapiens
<400> 5
   aagaggagga aggaagatgc gagaaggcag aggaggaggg agggagggaa ggagcgcgga 60
   gcccggcccg gaagctagga gccattccgt agtgccatcc cgagcaacgc actgctgcag 120
   cttccctgag cctttccagc aagtttgttc aagattggct gtcaagaatc atggactgtt 180
   attatatgcc ttgttttctg tcaagacacc atgattcctg gtaaccgaat gctgatggtc 240
   gttttattat gccaagtcct gctaggaggc gcgagccatg ctagtttgat acctgagacg 300
   gggaagaaaa aagtcgccga gattcagggc cacgcgggag gacgccgctc agggcagagc 360
   catgagctcc tgcgggactt cgaggcgaca cttctgcaga tgtttgggct gcgccgccgc 420
   ccgcagccta gcaagagtgc cgtcattccg gactacatgc gggatcttta ccggcttcag 480
   tctggggagg aggaggaaga gcagatccac agcactggtc ttgagtatcc tgagcgcccg 540
   gccagccggg ccaacaccgt gaggagcttc caccacgaag aacatctgga gaacatccca 600
   gggaccagtg aaaactctgc ttttcgtttc ctctttaacc tcagcagcat ccctgagaac 660
   gaggtgatct cctctgcaga gcttcggctc ttccgggagc aggtggacca gggccctgat 720
   tgggaaaggg gcttccaccg tataaacatt tatgaggtta tgaagccccc agcagaagtg 780
   gtgcctgggc acctcatcac acgactactg gacacgagac tggtccacca caatgtgaca 840
   cggtgggaaa cttttgatgt gagccctgcg gtccttcgct ggacccggga gaagcagcca 900
   aactatgggc tagccattga ggtgactcac ctccatcaga ctcggaccca ccagggccag 960
   catgtcagga ttagccgatc gttacctcaa gggagtggga attgggccca gctccggccc 1020
   ctcctggtca cctttggcca tgatggccgg ggccatgcct tgacccgacg ccggagggcc 1080
   aagcgtagcc ctaagcatca ctcacagcgg gccaggaaga agaataagaa ctgccggcgc 1140
   cactcgctct atgtggactt cagcgatgtg ggctggaatg actggattgt ggccccacca 1200
   ggctaccagg ccttctactg ccatggggac tgcccctttc cactggctga ccacctcaac 1260
   tcaaccaacc atgccattgt gcagaccctg gtcaattctg tcaattccag tatccccaaa 1320
   gcctgttgtg tgcccactga actgagtgcc atctccatgc tgtacctgga tgagtatgat 1380
   aaggtggtac tgaaaaatta tcaggagatg gtagtagagg gatgtgggtg ccgctgagat 1440
   caggcagtcc ttgaggatag acagatatac acaccacaca cacacaccac atacaccaca 1500
   cacacacgtt cccatccact cacccacaca ctacacagac tgcttcctta tagctggact 1560
   tttatttaaa aaaaaaaaaa aaaaaggaaa aaatccctaa acattcacct tgaccttatt 1620
   tatgacttta cgtgcaaatg ttttgaccat attgatcata tattttgaca aaatatattt 1680
   ataactacgt attaaaagaa aaaaataaaa tgagtcatta ttttaaaggt aaaaaaaaaa 1740
   aaaaaaaa 1748
<210> 6
   <211> 1802
   <212> DNA
   <213> Homo sapiens
<400> 6
   gaaggaagtg gcgggggaag gagtgtggtg gtggtttaaa aaataaggga agccgaggcg      60
   agagagacgc agacgcagag gtcgagcgca ggccgaaagc tgttcaccgt tttctcgact     120
   ccggggaaca tggagccatt ccgtagtgcc atcccgagca acgcactgct gcagcttccc     180
   tgagcctttc cagcaagttt gttcaagatt ggctgtcaag aatcatggac tgttattata     240
   tgccttgttt tctgtcaaga caccatgatt cctggtaacc gaatgctgat ggtcgtttta     300
   ttatgccaag tcctgctagg aggcgcgagc catgctagtt tgatacctga gacggggaag     360
   aaaaaagtcg ccgagattca gggccacgcg ggaggacgcc gctcagggca gagccatgag     420
   ctcctgcggg acttcgaggc gacacttctg cagatgtttg ggctgcgccg ccgcccgcag     480
   cctagcaaga gtgccgtcat tccggactac atgcgggatc tttaccggct tcagtctggg     540
   gaggaggagg aagagcagat ccacagcact ggtcttgagt atcctgagcg cccggccagc     600
   cgggccaaca ccgtgaggag cttccaccac gaagaacatc tggagaacat cccagggacc     660
   agtgaaaact ctgcttttcg tttcctcttt aacctcagca gcatccctga gaacgaggtg     720
   atctcctctg cagagcttcg gctcttccgg gagcaggtgg accagggccc tgattgggaa     780
   aggggcttcc accgtataaa catttatgag gttatgaagc ccccagcaga agtggtgcct     840
   gggcacctca tcacacgact actggacacg agactggtcc accacaatgt gacacggtgg     900
   gaaacttttg atgtgagccc tgcggtcctt cgctggaccc gggagaagca gccaaactat     960
   gggctagcca ttgaggtgac tcacctccat cagactcgga cccaccaggg ccagcatgtc    1020
   aggattagcc gatcgttacc tcaagggagt gggaattggg cccagctccg gcccctcctg    1080
   gtcacctttg gccatgatgg ccggggccat gccttgaccc gacgccggag ggccaagcgt    1140
   agccctaagc atcactcaca gcgggccagg aagaagaata agaactgccg gcgccactcg    1200
   ctctatgtgg acttcagcga tgtgggctgg aatgactgga ttgtggcccc accaggctac    1260
   caggccttct actgccatgg ggactgcccc tttccactgg ctgaccacct caactcaacc    1320
   aaccatgcca ttgtgcagac cctggtcaat tctgtcaatt ccagtatccc caaagcctgt    1380
   tgtgtgccca ctgaactgag tgccatctcc atgctgtacc tggatgagta tgataaggtg    1440
   gtactgaaaa attatcagga gatggtagta gagggatgtg ggtgccgctg agatcaggca    1500
   gtccttgagg atagacagat atacacacca cacacacaca ccacatacac cacacacaca    1560
   cgttcccatc cactcaccca cacactacac agactgcttc cttatagctg gacttttatt    1620
   taaaaaaaaa aaaaaaaaag gaaaaaatcc ctaaacattc accttgacct tatttatgac    1680
   tttacgtgca aatgttttga ccatattgat catatatttt gacaaaatat atttataact    1740
   acgtattaaa agaaaaaaat aaaatgagtc attattttaa aggtaaaaaa aaaaaaaaaa    1800
   aa                                                                   1802
<210> 7
   <211> 408
   <212> PRT
   <213> Homo sapiens
<400> 7
<210> 8
   <211> 2207
   <212> DNA
   <213> Homo sapiens
<400> 8
   ctcttgaaga gggctggtat atttgtgcct gctggaggtg gaattaacag taagaaggag    60
   aaagggattg aatggactta caggaaggat ttcaagtaaa ttcagggaaa cacatttact   120
   tgaatagtac aacctagagt attattttac actaagacga cacaaaagat gttaaagtta   180
   tcaccaagct gccggacaga tatatattcc aacaccaagg tgcagatcag catagatctg   240
   tgattcagaa atcaggattt gttttggaaa gagctcaagg gttgagaaga actcaaaagc   300
   aagtgaagat tactttggga actacagttt atcagaagat caacttttgc taattcaaat   360
   accaaaggcc tgattatcat aaattcatat aggaatgcat aggtcatctg atcaaataat   420
   attagccgtc ttctgctaca tcaatgcagc aaaaactctt aacaactgtg gataattgga   480
   aatctgagtt tcagctttct tagaaataac tactcttgac atattccaaa atatttaaaa 540
   taggacagga aaatcggtga ggatgttgtg ctcagaaatg tcactgtcat gaaaaatagg 600
   taaatttgtt ttttcagcta ctgggaaact gtacctccta gaaccttagg tttttttttt 660
   ttttaagagg acaagaagga ctaaaaatat caacttttgc ttttggacaa aaatgcatct 720
   gactgtattt ttacttaagg gtattgtggg tttcctctgg agctgctggg ttctagtggg 780
   ttatgcaaaa ggaggtttgg gagacaatca tgttcactcc agttttattt atagaagact 840
   acggaaccac gaaagacggg aaatacaaag ggaaattctc tctatcttgg gtttgcctca 900
   cagacccaga ccattttcac ctggaaaaca agcgtcctct gcacctctct ttatgctgga 960
   tctctacaat gccatgacca atgaagaaaa tcctgaagag tcggagtact cagtaagggc 1020
   atccttggca gaagagacca gaggggcaag aaagggatac ccagcctctc ccaatgggta 1080
   tcctcgtcgc atacagttat ctcggacgac tcctctgacc acccagagtc ctcctctagc 1140
   cagcctccat gataccaact ttctgaatga tgctgacatg gtcatgagct ttgtcaactt 1200
   agttgaaaga gacaaggatt tttctcacca gcgaaggcat tacaaagaat ttcgatttga 1260
   tcttacccaa attcctcatg gagaggcagt gacagcagct gaattccgga tatacaagga 1320
   ccggagcaac aaccgatttg aaaatgaaac aattaagatt agcatatatc aaatcatcaa 1380
   ggaatacaca aatagggatg cagatctgtt cttgttagac acaagaaagg cccaagcttt 1440
   agatgtgggt tggcttgtct ttgatatcac tgtgaccagc aatcattggg tgattaatcc 1500
   ccagaataat ttgggcttac agctctgtgc agaaacaggg gatggacgca gtatcaacgt 1560
   aaaatctgct ggtcttgtgg gaagacaggg acctcagtca aaacaaccat tcatggtggc 1620
   cttcttcaag gcgagtgagg tacttcttcg atccgtgaga gcagccaaca aacgaaaaaa 1680
   tcaaaaccgc aataaatcca gctctcatca ggactcctcc agaatgtcca gtgttggaga 1740
   ttataacaca agtgagcaaa aacaagcctg taagaagcac gaactctatg tgagcttccg 1800
   ggatctggga tggcaggact ggattatagc accagaagga tacgctgcat tttattgtga 1860
   tggagaatgt tcttttccac ttaacgccca tatgaatgcc accaaccacg ctatagttca 1920
   gactctggtt catctgatgt ttcctgacca cgtaccaaag ccttgttgtg ctccaaccaa 1980
   attaaatgcc atctctgttc tgtactttga tgacagctcc aatgtcattt tgaaaaaata 2040
   tagaaatatg gtagtacgct catgtggctg ccactaatat taaataatat tgataataac 2100
   aaaaagatct gtattaaggt ttatggctgc aataaaaagc atactttcag acaaacgggg 2160
   aatttcctaa aaaaaaaaaa aaaaaaaaaa aaaaaaaaaa aaaaaaa 2207
<210> 9
   <211> 513
   <212> PRT
   <213> Homo sapiens
<400> 9
<210> 10
   <211> 3105
   <212> DNA
   <213> Homo sapiens
<400> 10
   caactggggg cgccccggac gaccatgaga gataaggact gagggccagg aaggggaagc      60
   gagcccgccg agaggtggcg gggactgctc acgccaaggg ccacagcggc cgcgctccgg 120
   cctcgctccg ccgctccacg cctcgcggga tccgcggggg cagcccggcc gggcggggat 180
   gccggggctg gggcggaggg cgcagtggct gtgctggtgg tgggggctgc tgtgcagctg 240
   ctgcgggccc ccgccgctgc ggccgccctt gcccgctgcc gcggccgccg ccgccggggg 300
   gcagctgctg ggggacggcg ggagccccgg ccgcacggag cagccgccgc cgtcgccgca 360
   gtcctcctcg ggcttcctgt accggcggct caagacgcag gagaagcggg agatgcagaa 420
   ggagatcttg tcggtgctgg ggctcccgca ccggccccgg cccctgcacg gcctccaaca 480
   gccgcagccc ccggcgctcc ggcagcagga ggagcagcag cagcagcagc agctgcctcg 540
   cggagagccc cctcccgggc gactgaagtc cgcgcccctc ttcatgctgg atctgtacaa 600
   cgccctgtcc gccgacaacg acgaggacgg ggcgtcggag ggggagaggc agcagtcctg 660
   gccccacgaa gcagccagct cgtcccagcg tcggcagccg cccccgggcg ccgcgcaccc 720
   gctcaaccgc aagagccttc tggcccccgg atctggcagc ggcggcgcgt ccccactgac 780
   cagcgcgcag gacagcgcct tcctcaacga cgcggacatg gtcatgagct ttgtgaacct 840
   ggtggagtac gacaaggagt tctcccctcg tcagcgacac cacaaagagt tcaagttcaa 900
   cttatcccag attcctgagg gtgaggtggt gacggctgca gaattccgca tctacaagga 960
   ctgtgttatg ggagttttta aaaaccaaac ttttcttatc agcatttatc aagtcttaca 1020
   ggagcatcag cacagagact ctgacctgtt tttgttggac acccgtgtag tatgggcctc 1080
   agaagaaggc tggctggaat ttgacatcac ggccactagc aatctgtggg ttgtgactcc 1140
   acagcataac atggggcttc agctgagcgt ggtgacaagg gatggagtcc acgtccaccc 1200
   ccgagccgca ggcctggtgg gcagagacgg cccttacgac aagcagccct tcatggtggc 1260
   tttcttcaaa gtgagtgagg tgcacgtgcg caccaccagg tcagcctcca gccggcgccg 1320
   acaacagagt cgtaatcgct ctacccagtc ccaggacgtg gcgcgggtct ccagtgcttc 1380
   agattacaac agcagtgaat tgaaaacagc ctgcaggaag catgagctgt atgtgagttt 1440
   ccaagacctg ggatggcagg actggatcat tgcacccaag ggctatgctg ccaattactg 1500
   tgatggagaa tgctccttcc cactcaacgc acacatgaat gcaaccaacc acgcgattgt 1560
   gcagaccttg gttcacctta tgaaccccga gtatgtcccc aaaccgtgct gtgcgccaac 1620
   taagctaaat gccatctcgg ttctttactt tgatgacaac tccaatgtca ttctgaaaaa 1680
   atacaggaat atggttgtaa gagcttgtgg atgccactaa ctcgaaacca gatgctgggg 1740
   acacacattc tgccttggat tcctagatta catctgcctt aaaaaaacac ggaagcacag 1800
   ttggaggtgg gacgatgaga ctttgaaact atctcatgcc agtgccttat tacccaggaa 1860
   gattttaaag gacctcatta ataatttgct cacttggtaa atgacgtgag tagttgttgg 1920
   tctgtagcaa gctgagtttg gatgtctgta gcataaggtc tggtaactgc agaaacataa 1980
   ccgtgaagct cttcctaccc tcctccccca aaaacccacc aaaattagtt ttagctgtag 2040
   atcaagctat ttggggtgtt tgttagtaaa tagggaaaat aatctcaaag gagttaaatg 2100
   tattcttggc taaaggatca gctggttcag tactgtctat caaaggtaga ttttacagag 2160
   aacagaaatc ggggaagtgg ggggaacgcc tctgttcagt tcattcccag aagtccacag 2220
   gacgcacagc ccaggccaca gccagggctc cacggggcgc ccttgtctca gtcattgctg 2280
   ttgtatgttc gtgctggagt tttgttggtg tgaaaataca cttatttcag ccaaaacata 2340
   ccatttctac acctcaatcc tccatttgct gtactctttg ctagtaccaa aagtagactg 2400
   attacactga ggtgaggcta caaggggtgt gtaaccgtgt aacacgtgaa ggcaatgctc 2460
   acctcttctt taccagaacg gttctttgac cagcacatta acttctggac tgccggctct 2520
   agtacctttt cagtaaagtg gttctctgcc tttttactat acagcatacc acgccacagg 2580
   gttagaacca acgaagaaaa taaaatgagg gtgcccagct tataagaatg gtgttagggg 2640
   gatgagcatg ctgtttatga acggaaatca tgatttccct tgtagaaagt gaggctcaga 2700
   ttaaatttta gaatattttc taaatgtctt tttcacaatc atgtactggg aaggcaattt 2760
   catactaaac tgattaaata atacatttat aatctacaac tgtttgcact tacagctttt 2820
   tttgtaaata taaactataa tttattgtct attttatatc tgttttgctg taacattgaa 2880
   ggaaagacca gacttttaaa aaaaaagagt ttatttagaa agtatcatag tgtaaacaaa 2940
   caaattgtac cactttgatt ttcttggaat acaagactcg tgatgcaaag ctgaagttgt 3000
   gtgtacaaga ctcttgacag ttgtgcttct ctaggaggtt gggttttttt aaaaaaagaa 3060
   ttatctgtga accatacgtg attaataaag atttccttta aggca 3105
<210> 11
   <211> 431
   <212> PRT
   <213> Homo sapiens
<400> 11
   Met His Val Arg Ser Leu Arg Ala Ala Ala Pro His Ser Phe Val Ala
   1 5 10 15
   Leu Trp Ala Pro Leu Phe Leu Leu Arg Ser Ala Leu Ala Asp Phe Ser
   20 25 30
   Leu Asp Asn Glu Val His Ser Ser Phe Ile His Arg Arg Leu Arg Ser
   35 40 45
   Gln Glu Arg Arg Glu Met Gln Arg Glu Ile Leu Ser Ile Leu Gly Leu
   50 55 60
   Pro His Arg Pro Arg Pro His Leu Gln Gly Lys His Asn Ser Ala Pro
   65 70 75 80
   Met Phe Met Leu Asp Leu Tyr Asn Ala Met Ala Val Glu Glu Gly Gly
   85 90 95
   Gly Pro Gly Gly Gln Gly Phe Ser Tyr Pro Tyr Lys Ala Val Phe Ser
   100 105 110
   Thr Gln Gly Pro Pro Leu Ala Ser Leu Gln Asp Ser His Phe Leu Thr
           115                 120                 125
   Asp Ala Asp Met Val Met Ser Phe Val Asn Leu Val Glu His Asp Lys
       130                 135                 140
   Glu Phe Phe His Pro Arg Tyr His His Arg Glu Phe Arg Phe Asp Leu
   145                 150                 155                 160
   Ser Lys Ile Pro Glu Gly Glu Ala Val Thr Ala Ala Glu Phe Arg Ile
                   165                 170                 175
   Tyr Lys Asp Tyr Ile Arg Glu Arg Phe Asp Asn Glu Thr Phe Arg Ile
               180                 185                 190
   Ser Val Tyr Gln Val Leu Gln Glu His Leu Gly Arg Glu Ser Asp Leu
           195                 200                 205
   Phe Leu Leu Asp Ser Arg Thr Leu Trp Ala Ser Glu Glu Gly Trp Leu
       210                 215                 220
   Val Phe Asp Ile Thr Ala Thr Ser Asn His Trp Val Val Asn Pro Arg
   225                 230                 235                 240
   His Asn Leu Gly Leu Gln Leu Ser Val Glu Thr Leu Asp Gly Gln Ser
                   245                 250                 255
   Ile Asn Pro Lys Leu Ala Gly Leu Ile Gly Arg His Gly Pro Gln Asn
               260                 265                 270
   Lys Gln Pro Phe Met Val Ala Phe Phe Lys Ala Thr Glu Val His Phe
           275                 280                 285
   Arg Ser Ile Arg Ser Thr Gly Ser Lys Gln Arg Ser Gln Asn Arg Ser
       290                 295                 300
   Lys Thr Pro Lys Asn Gln Glu Ala Leu Arg Met Ala Asn Val Ala Glu
   305                 310                 315                 320
   Asn Ser Ser Ser Asp Gln Arg Gln Ala Cys Lys Lys His Glu Leu Tyr
                   325                 330                 335
   Val Ser Phe Arg Asp Leu Gly Trp Gln Asp Trp Ile Ile Ala Pro Glu
               340                 345                 350
   Gly Tyr Ala Ala Tyr Tyr Cys Glu Gly Glu Cys Ala Phe Pro Leu Asn
           355                 360                 365
   Ser Tyr Met Asn Ala Thr Asn His Ala Ile Val Gln Thr Leu Val His
       370                 375                 380
   Phe Ile Asn Pro Glu Thr Val Pro Lys Pro Cys Cys Ala Pro Thr Gln
   385 390 395 400
   Leu Asn Ala Ile Ser Val Leu Tyr Phe Asp Asp Ser Ser Asn Val Ile
   405 410 415
   Leu Lys Lys Tyr Arg Asn Met Val Val Arg Ala Cys Gly Cys His
   420 425 430
<210> 12
   <211> 1896
   <212> DNA
   <213> Homo sapiens
<400> 12
   gggcgcagcg gggcccgtct gcagcaagtg accgacggcc gggacggccg cctgccccct     60
   ctgccacctg gggcggtgcg ggcccggagc ccggagcccg ggtagcgcgt agagccggcg    120
   cgatgcacgt gcgctcactg cgagctgcgg cgccgcacag cttcgtggcg ctctgggcac    180
   ccctgttcct gctgcgctcc gccctggccg acttcagcct ggacaacgag gtgcactcga    240
   gcttcatcca ccggcgcctc cgcagccagg agcggcggga gatgcagcgc gagatcctct    300
   ccattttggg cttgccccac cgcccgcgcc cgcacctcca gggcaagcac aactcggcac    360
   ccatgttcat gctggacctg tacaacgcca tggcggtgga ggagggcggc gggcccggcg    420
   gccagggctt ctcctacccc tacaaggccg tcttcagtac ccagggcccc cctctggcca    480
   gcctgcaaga tagccatttc ctcaccgacg ccgacatggt catgagcttc gtcaacctcg    540
   tggaacatga caaggaattc ttccacccac gctaccacca tcgagagttc cggtttgatc    600
   tttccaagat cccagaaggg gaagctgtca cggcagccga attccggatc tacaaggact    660
   acatccggga acgcttcgac aatgagacgt tccggatcag cgtttatcag gtgctccagg    720
   agcacttggg cagggaatcg gatctcttcc tgctcgacag ccgtaccctc tgggcctcgg    780
   aggagggctg gctggtgttt gacatcacag ccaccagcaa ccactgggtg gtcaatccgc    840
   ggcacaacct gggcctgcag ctctcggtgg agacgctgga tgggcagagc atcaacccca    900
   agttggcggg cctgattggg cggcacgggc cccagaacaa gcagcccttc atggtggctt    960
   tcttcaaggc cacggaggtc cacttccgca gcatccggtc cacggggagc aaacagcgca   1020
   gccagaaccg ctccaagacg cccaagaacc aggaagccct gcggatggcc aacgtggcag   1080
   agaacagcag cagcgaccag aggcaggcct gtaagaagca cgagctgtat gtcagcttcc   1140
   gagacctggg ctggcaggac tggatcatcg cgcctgaagg ctacgccgcc tactactgtg   1200
   agggggagtg tgccttccct ctgaactcct acatgaacgc caccaaccac gccatcgtgc   1260
   agacgctggt ccacttcatc aacccggaaa cggtgcccaa gccctgctgt gcgcccacgc   1320
   agctcaatgc catctccgtc ctctacttcg atgacagctc caacgtcatc ctgaagaaat   1380
   acagaaacat ggtggtccgg gcctgtggct gccactagct cctccgagaa ttcagaccct   1440
   ttggggccaa gtttttctgg atcctccatt gctcgccttg gccaggaacc agcagaccaa   1500
   ctgccttttg tgagaccttc ccctccctat ccccaacttt aaaggtgtga gagtattagg   1560
   aaacatgagc agcatatggc ttttgatcag tttttcagtg gcagcatcca atgaacaaga   1620
   tcctacaagc tgtgcaggca aaacctagca ggaaaaaaaa acaacgcata aagaaaaatg   1680
   gccgggccag gtcattggct gggaagtctc agccatgcac ggactcgttt ccagaggtaa   1740
   ttatgagcgc ctaccagcca ggccacccag ccgtgggagg aagggggcgt ggcaaggggt   1800
   gggcacattg gtgtctgtgc gaaaggaaaa ttgacccgga agttcctgta ataaatgtca   1860
   caataaaacg aatgaatgaa aaaaaaaaaa aaaaaa                              1896
<210> 13
   <211> 501
   <212> PRT
   <213> Homo sapiens
<400> 13
   Met Arg Leu Pro Lys Leu Leu Thr Phe Leu Leu Trp Tyr Leu Ala Trp
   1               5                   10                  15
   Leu Asp Leu Glu Phe Ile Cys Thr Val Leu Gly Ala Pro Asp Leu Gly
               20                  25                  30
   Gln Arg Pro Gln Gly Thr Arg Pro Gly Leu Ala Lys Ala Glu Ala Lys
           35                  40                  45
   Glu Arg Pro Pro Leu Ala Arg Asn Val Phe Arg Pro Gly Gly His Ser
       50                  55                  60
   Tyr Gly Gly Gly Ala Thr Asn Ala Asn Ala Arg Ala Lys Gly Gly Thr
   65                  70                  75                  80
   Gly Gln Thr Gly Gly Leu Thr Gln Pro Lys Lys Asp Glu Pro Lys Lys
                   85                  90                  95
   Leu Pro Pro Arg Pro Gly Gly Pro Glu Pro Lys Pro Gly His Pro Pro
               100                 105                 110
   Gln Thr Arg Gln Ala Thr Ala Arg Thr Val Thr Pro Lys Gly Gln Leu
           115                 120                 125
   Pro Gly Gly Lys Ala Pro Pro Lys Ala Gly Ser Val Pro Ser Ser Phe
       130                 135                 140
   Leu Leu Lys Lys Ala Arg Glu Pro Gly Pro Pro Arg Glu Pro Lys Glu
   145                 150                 155                 160
   Pro Phe Arg Pro Pro Pro Ile Thr Pro His Glu Tyr Met Leu Ser Leu
                   165                 170                 175
   Tyr Arg Thr Leu Ser Asp Ala Asp Arg Lys Gly Gly Asn Ser Ser Val
               180                 185                 190
   Lys Leu Glu Ala Gly Leu Ala Asn Thr Ile Thr Ser Phe Ile Asp Lys
   195 200 205
   Gly Gln Asp Asp Arg Gly Pro Val Val Arg Lys Gln Arg Tyr Val Phe
   210 215 220
   Asp Ile Ser Ala Leu Glu Lys Asp Gly Leu Leu Gly Ala Glu Leu Arg
   225 230 235 240
   Ile Leu Arg Lys Lys Pro Ser Asp Thr Ala Lys Pro Ala Ala Pro Gly
   245 250 255
   Gly Gly Arg Ala Ala Gln Leu Lys Leu Ser Ser Cys Pro Ser Gly Arg
   260 265 270
   Gln Pro Ala Ser Leu Leu Asp Val Arg Ser Val Pro Gly Leu Asp Gly
   275 280 285
   Ser Gly Trp Glu Val Phe Asp Ile Trp Lys Leu Phe Arg Asn Phe Lys
   290 295 300
   Asn Ser Ala Gln Leu Cys Leu Glu Leu Glu Ala Trp Glu Arg Gly Arg
   305 310 315 320
   Ala Val Asp Leu Arg Gly Leu Gly Phe Asp Arg Ala Ala Arg Gln Val
   325 330 335
   His Glu Lys Ala Leu Phe Leu Val Phe Gly Arg Thr Lys Lys Arg Asp
   340 345 350
   Leu Phe Phe Asn Glu Ile Lys Ala Arg Ser Gly Gln Asp Asp Lys Thr
   355 360 365
   Val Tyr Glu Tyr Leu Phe Ser Gln Arg Arg Lys Arg Arg Ala Pro Leu
   370 375 380
   Ala Thr Arg Gln Gly Lys Arg Pro Ser Lys Asn Leu Lys Ala Arg Cys
   385 390 395 400
   Ser Arg Lys Ala Leu His Val Asn Phe Lys Asp Met Gly Trp Asp Asp
   405 410 415
   Trp Ile Ile Ala Pro Leu Glu Tyr Glu Ala Phe His Cys Glu Gly Leu
   420 425 430
   Cys Glu Phe Pro Leu Arg Ser His Leu Glu Pro Thr Asn His Ala Val
   435 440 445
   Ile Gln Thr Leu Met Asn Ser Met Asp Pro Glu Ser Thr Pro Pro Thr
   450 455 460
   Cys Cys Val Pro Thr Arg Leu Ser Pro Ile Ser Ile Leu Phe Ile Asp
   465 470 475 480
   Ser Ala Asn Asn Val Val Tyr Lys Gln Tyr Glu Asp Met Val Val Glu
   485 490 495
   Ser Cys Gly Cys Arg
   500
<210> 14
   <211> 2383
   <212> DNA
   <213> Homo sapiens
<400> 14
   ctccttcaag ccctcagtca gttgtgcagg agaaaggggg cggttggctt tctcctttca 60
   agaacgagtt attttcagct gctgactgga gacggtgcac gtctggatac gagagcattt 120
   ccactatggg actggataca aacacacacc cggcagactt caagagtctc agactgagga 180
   gaaagccttt ccttctgctg ctactgctgc tgccgctgct tttgaaagtc cactcctttc 240
   atggtttttc ctgccaaacc agaggcacct ttgctgctgc cgctgttctc tttggtgtca 300
   ttcagcggct ggccagagga tgagactccc caaactcctc actttcttgc tttggtacct 360
   ggcttggctg gacctggaat tcatctgcac tgtgttgggt gcccctgact tgggccagag 420
   accccagggg accaggccag gattggccaa agcagaggcc aaggagaggc cccccctggc 480
   ccggaacgtc ttcaggccag ggggtcacag ctatggtggg ggggccacca atgccaatgc 540
   cagggcaaag ggaggcaccg ggcagacagg aggcctgaca cagcccaaga aggatgaacc 600
   caaaaagctg ccccccagac cgggcggccc tgaacccaag ccaggacacc ctccccaaac 660
   aaggcaggct acagcccgga ctgtgacccc aaaaggacag cttcccggag gcaaggcacc 720
   cccaaaagca ggatctgtcc ccagctcctt cctgctgaag aaggccaggg agcccgggcc 780
   cccacgagag cccaaggagc cgtttcgccc accccccatc acaccccacg agtacatgct 840
   ctcgctgtac aggacgctgt ccgatgctga cagaaaggga ggcaacagca gcgtgaagtt 900
   ggaggctggc ctggccaaca ccatcaccag ctttattgac aaagggcaag atgaccgagg 960
   tcccgtggtc aggaagcaga ggtacgtgtt tgacattagt gccctggaga aggatgggct 1020
   gctgggggcc gagctgcgga tcttgcggaa gaagccctcg gacacggcca agccagcggc 1080
   ccccggaggc gggcgggctg cccagctgaa gctgtccagc tgccccagcg gccggcagcc 1140
   ggcctccttg ctggatgtgc gctccgtgcc aggcctggac ggatctggct gggaggtgtt 1200
   cgacatctgg aagctcttcc gaaactttaa gaactcggcc cagctgtgcc tggagctgga 1260
   ggcctgggaa cggggcaggg ccgtggacct ccgtggcctg ggcttcgacc gcgccgcccg 1320
   gcaggtccac gagaaggccc tgttcctggt gtttggccgc accaagaaac gggacctgtt 1380
   ctttaatgag attaaggccc gctctggcca ggacgataag accgtgtatg agtacctgtt 1440
   cagccagcgg cgaaaacggc gggccccact ggccactcgc cagggcaagc gacccagcaa 1500
   gaaccttaag gctcgctgca gtcggaaggc actgcatgtc aacttcaagg acatgggctg 1560
   ggacgactgg atcatcgcac cccttgagta cgaggctttc cactgcgagg ggctgtgcga 1620
   gttcccattg cgctcccacc tggagcccac gaatcatgca gtcatccaga ccctgatgaa 1680
   ctccatggac cccgagtcca caccacccac ctgctgtgtg cccacgcggc tgagtcccat 1740
   cagcatcctc ttcattgact ctgccaacaa cgtggtgtat aagcagtatg aggacatggt 1800
   cgtggagtcg tgtggctgca ggtagcagca ctggccctct gtcttcctgg gtggcacatc 1860
   ccaagagccc cttcctgcac tcctggaatc acagaggggt caggaagctg tggcaggagc 1920
   atctacacag cttgggtgaa aggggattcc aataagcttg ctcgctctct gagtgtgact 1980
   tgggctaaag gccccctttt atccacaagt tcccctggct gaggattgct gcccgtctgc 2040
   tgatgtgacc agtggcaggc acaggtccag ggagacagac tctgaatggg actgagtccc 2100
   aggaaacagt gctttccgat gagactcagc ccaccatttc tcctcacctg ggccttctca 2160
   gcctctggac tctcctaagc acctctcagg agagccacag gtgccactgc ctcctcaaat 2220
   cacatttgtg cctggtgact tcctgtccct gggacagttg agaagctgac tgggcaagag 2280
   tgggagagaa gaggagaggg cttggataga gttgaggagt gtgaggctgt tagactgtta 2340
   gatttaaatg tatattgatg agataaaaag caaaactgtg cct 2383
<210> 15
   <211> 455
   <212> PRT
   <213> Homo sapiens
<400> 15
<210> 16
   <211> 3716
   <212> DNA
   <213> Homo sapiens
<400> 16
   cccgaggagc cgggccccgg ccgctgtcca gccgctccgt gccccgcgcg tcctgcgccg 60
   ccgccaccgc ctcctgggga gacgcagcca cttgcccgcc atggatactc ccagggtcct 120
   gctctcggcc gtcttcctca tcagttttct gtgggatttg cccggtttcc agcaggcttc 180
   catctcatcc tcctcgtcgt ccgccgagct gggttccacc aagggcatgc gaagccgcaa 240
   ggaaggcaag atgcagcggg cgccgcgcga cagtgacgcg ggccgggagg gccaggaacc 300
   acagccgcgg cctcaggacg aaccccgggc tcagcagccc cgggcgcagg agccgccagg 360
   caggggtccg cgcgtggtgc cccacgagta catgctgtca atctacagga cttactccat 420
   cgctgagaag ctgggcatca atgccagctt tttccagtct tccaagtcgg ctaatacgat 480
   caccagcttt gtagacaggg gactagacga tctctcgcac actcctctcc ggagacagaa 540
   gtatttgttt gatgtgtcca tgctctcaga caaagaagag ctggtgggcg cggagctgcg 600
   gctctttcgc caggcgccct cagcgccctg ggggccacca gccgggccgc tccacgtgca 660
   gctcttccct tgcctttcgc ccctactgct ggacgcgcgg accctggacc cgcagggggc 720
   gccgccggcc ggctgggaag tcttcgacgt gtggcagggc ctgcgccacc agccctggaa 780
   gcagctgtgc ttggagctgc gggccgcatg gggcgagctg gacgccgggg aggccgaggc 840
   gcgcgcgcgg ggaccccagc aaccgccgcc cccggacctg cggagtctgg gcttcggccg 900
   gagggtgcgg cctccccagg agcgggccct gctggtggta ttcaccagat cccagcgcaa 960
   gaacctgttc gcagagatgc gcgagcagct gggctcggcc gaggctgcgg gcccgggcgc 1020
   gggcgccgag gggtcgtggc cgccgccgtc gggcgccccg gatgccaggc cttggctgcc 1080
   ctcgcccggc cgccggcggc ggcgcacggc cttcgccagt cgccatggca agcggcacgg 1140
   caagaagtcc aggctacgct gcagcaagaa gcccctgcac gtgaacttca aggagctggg 1200
   ctgggacgac tggattatcg cgcccctgga gtacgaggcc tatcactgcg agggtgtatg 1260
   cgacttcccg ctgcgctcgc acctggagcc caccaaccac gccatcatcc agacgctgat 1320
   gaactccatg gaccccggct ccaccccgcc cagctgctgc gtgcccacca aattgactcc 1380
   catcagcatt ctatacatcg acgcgggcaa taatgtggtc tacaagcagt acgaggacat 1440
   ggtggtggag tcgtgcggct gcaggtagcg gtgcctttcc cgccgccttg gcccggaacc 1500
   aaggtgggcc aaggtccgcc ttgcagggga ggcctggctg cagagaggcg gaggaggaag 1560
   ctggcgctgg gggaggctga gggtgaggga acagcctgga tgtgagagcc ggtgggagag 1620
   aagggagcgc agccttccca gtaacttcta cctgccagcc cagagggaaa tatggatttt 1680
   cacaccttgc ctggccaccc tggaaaaaca agccaaggag gatttctttt gttctgtttt 1740
   ctctctctct ctctctctct ctctctctct ctctctctct ctctctatta ctgtggcttt 1800
   ggatttcctt atgtgtctta caggctttga tagaagggga ggggaggaga gatgcatacc 1860
   cgtttctcaa ctgctccatg gattgaaaaa ataacagttt aaaaagggaa acaatgtggg 1920
   aggaagaatc accgttgacg catcttgatt tggttggttt ttacatgtgt aaagaaggtg 1980
   gggtctctgg ccatgtcata gcccatgtct tgtgccctcc cacacagaaa gtgttagata 2040
   gggaaattgg caaaaagaat agttaagtca ggaatggtcc tgcctataga agagctttga 2100
   gagaggtggg cccacgggtg cccctctcac ccatttgtgt actctgtgag tttaccagct 2160
   ctgccctggc ctctttcggt accaggaact ggcaaccttc atctcactcc tgagggccca 2220
   ggtctctgcc ttcattgttg ctttttctgg tgggggcaag gggagctggt atggatggaa 2280
   tgacaagaat tagtccaaat ggaacccctt gaaggataat gagaaaccac aaggcctgcc 2340
   tctgactggg gctgacacgg aggtgcatta gcccaggctg gaggtagccc acccaaatgc 2400
   cctttctgat tctaattgat ttctttcaac agaatttgcc aaaattcaga catgcacttc 2460
   taaggggaag gtgattttcc agttcaaaaa aatgggcagg agtggggaac aaaacaatta 2520
   acgtaagagc tacaaaggag ggaaaaggaa ccaagaagta gaaggagtcc catcaggagg 2580
   gaagatggtg ggcctcaggg aggatgggga tcaagggaca ggccaggagc caggagtggg 2640
   gaagggaggg atgaaagggg acacaagtcc ctgtctctga agtttcttta aaatctgagt 2700
   tccctcccct ctctttgaca ttcctgaaag attaccagcc agcaatagcc cagggctccc 2760
   ccaaaagaat tggttcagat tgtaattatc agttaggcaa tgtttttaaa acttagtaat 2820
   gagaaactgt gaaaagagcc aagtgttaca ttgagcttgg ggtgggagat ggggaacagg 2880
   cagtgaggaa ggagacaggg gtggaattcg tcttctggga ggaagctgga gagagcacag 2940
   tgaaattgaa atacccattc ccagatagtc aaaaacatga actttccccc agcctgcacc 3000
   agtattgttt tcaaacattg cccatgagta ggccctttga agagttagct tcctcctcat 3060
   ctttgactat aaaattgttt aatcaatgga atttgtacca gccttttaaa aagttttagt 3120
   ttttcctaag tgattttgct ctcttccaat ctaaacctgt tgcttgtttg gttcagagaa 3180
   ctacaaactg tcaaagaaag ggtggggatg ataagaaatg ctaatataaa aatgctaagt 3240
   gaaaaaaaga cttggccagg agaaataatt taaaatgcac atttgctttg gatgcactgt 3300
   tgttctgtta aggctgtata tatttgttta tttaaggtga ctgaaagtgc aaagaggaaa 3360
   tggacagcat gcaattcatc ctaatgtaca aaacgttata tgcactcaaa tgttataatt 3420
   tctaatattt ttaaagttta tattcgagtt gtacaaagtt aagcattaat cagatatttc 3480
   attttttcat aatgttacca ttttcttaaa tattattaca aaattttaag tctgtctaat 3540
   ggagagtttt ttttaaactg tctacctcat ataatacaag tatttacaac gctaaagtta 3600
   ccagaggtca atgaataatc aaaacatttt ttacagtaca cctttcctgg atgatatgca 3660
   atcgaatgct atattattaa acgcattttt ctccttatta aaaaaaaaaa aaaaaa 3716
<210> 17
   <211> 450
   <212> PRT
   <213> Homo sapiens
<400> 17
   Met Asp Leu Ser Ala Ala Ala Ala Leu Cys Leu Trp Leu Leu Ser Ala
   1 5 10 15
   Cys Arg Pro Arg Asp Gly Leu Glu Ala Ala Ala Val Leu Arg Ala Ala
   20 25 30
   Gly Ala Gly Pro Val Arg Ser Pro Gly Gly Gly Gly Gly Gly Gly Gly
   35 40 45
   Gly Gly Arg Thr Leu Ala Gln Ala Ala Gly Ala Ala Ala Val Pro Ala
   50 55 60
   Ala Ala Val Pro Arg Ala Arg Ala Ala Arg Arg Ala Ala Gly Ser Gly
   65 70 75 80
   Phe Arg Asn Gly Ser Val Val Pro His His Phe Met Met Ser Leu Tyr
   85 90 95
   Arg Ser Leu Ala Gly Arg Ala Pro Ala Gly Ala Ala Ala Val Ser Ala
   100 105 110
   Ser Gly His Gly Arg Ala Asp Thr Ile Thr Gly Phe Thr Asp Gln Ala
   115 120 125
   Thr Gln Asp Glu Ser Ala Ala Glu Thr Gly Gln Ser Phe Leu Phe Asp
   130 135 140
   Val Ser Ser Leu Asn Asp Ala Asp Glu Val Val Gly Ala Glu Leu Arg
   145 150 155 160
   Val Leu Arg Arg Gly Ser Pro Glu Ser Gly Pro Gly Ser Trp Thr Ser
   165 170 175
   Pro Pro Leu Leu Leu Leu Ser Thr Cys Pro Gly Ala Ala Arg Ala Pro
   180 185 190
   Arg Leu Leu Tyr Ser Arg Ala Ala Glu Pro Leu Val Gly Gln Arg Trp
   195 200 205
   Glu Ala Phe Asp Val Ala Asp Ala Met Arg Arg His Arg Arg Glu Pro
   210 215 220
   Arg Pro Pro Arg Ala Phe Cys Leu Leu Leu Arg Ala Val Ala Gly Pro
   225 230 235 240
   Val Pro Ser Pro Leu Ala Leu Arg Arg Leu Gly Phe Gly Trp Pro Gly
   245 250 255
   Gly Gly Gly Ser Ala Ala Glu Glu Arg Ala Val Leu Val Val Ser Ser
   260 265 270
   Arg Thr Gln Arg Lys Glu Ser Leu Phe Arg Glu Ile Arg Ala Gln Ala
   275 280 285
   Arg Ala Leu Gly Ala Ala Leu Ala Ser Glu Pro Leu Pro Asp Pro Gly
   290 295 300
   Thr Gly Thr Ala Ser Pro Arg Ala Val Ile Gly Gly Arg Arg Arg Arg
   305 310 315 320
   Arg Thr Ala Leu Ala Gly Thr Arg Thr Ser Gln Gly Ser Gly Gly Gly
   325 330 335
   Ala Gly Arg Gly His Gly Arg Arg Gly Arg Ser Arg Cys Ser Arg Lys
   340 345 350
   Pro Leu His Val Asp Phe Lys Glu Leu Gly Trp Asp Asp Trp Ile Ile
   355 360 365
   Ala Pro Leu Asp Tyr Glu Ala Tyr His Cys Glu Gly Leu Cys Asp Phe
   370 375 380
   Pro Leu Arg Ser His Leu Glu Pro Thr Asn His Ala Ile Ile Gln Thr
   385 390 395 400
   Leu Leu Asn Ser Met Ala Pro Asp Ala Ala Pro Ala Ser Cys Cys Val
   405 410 415
   Pro Ala Arg Leu Ser Pro Ile Ser Ile Leu Tyr Ile Asp Ala Ala Asn
   420 425 430
   Asn Val Val Tyr Lys Gln Tyr Glu Asp Met Val Val Glu Ala Cys Gly
   435 440 445
   Cys Arg
   450
<210> 18
   <211> 1994
   <212> DNA
   <213> Homo sapiens
<400> 18
   ctttgaggcc gccgggagca tcctgtggcc tctctctgcg cggccacccg gccgcggcgc 60
   gaagcggtct ggagggcgag cccttccgcg gccccaactc tgccgccccg ttcccggcat 120
   tgggaaccag ggcagggagg gggcgggtgt ttctctgcgg gggagtgggg aggaagctgg 180
   gcgggtgcgc gcggtgcccc gagcctggaa ccacggaggg cgcgttggtc ttgggcggat 240
   ggagggggtg tcgcactgcc gcggggaggc gtgtcgggag gctggggcca gtggcagtcg 300
   cttggcgagg gtgggggcgt agcgctgcgg tgggaggagg cggctccggc cctggtctcc 360
   actctaggcc ggggtggggg gcgcatagcg gccgccggag ctttcagcag ggggcgctgc 420
   tccgggcgtt gggcgggggt ggggtgggcc aggagggggg gccgcgggct ggccgcgcac 480
   acttccccca ttattaaaca ctatgttcaa aaggcgccgg gggacttccc ggagccacgg 540
   agcccgcgcc gcccgcccgc ccggcccacg gagcccatgg acctgagcgc cgccgccgcg 600
   ctgtgccttt ggctgctgag cgcctgccgc ccccgcgacg ggctggaagc ggccgccgtg 660
   ctgcgagcgg cgggggctgg gccggtccgg agcccagggg gcggcggcgg cggcggcggc 720
   ggcgggcgga ctcttgccca ggctgcgggc gccgcggctg tcccggccgc cgcggttccc 780
   cgggcccgcg ccgcgcgccg cgccgcgggc tccggcttca ggaacggctc ggtggtgccg 840
   caccacttca tgatgtcgct ttaccggagc ctggccggga gggctccggc cggggcagcc 900
   gctgtctccg cctcggccca tggtcgcgcg gacacgatca ccggcttcac agaccaggcg 960
   acccaagacg aatcggcagc cgaaacaggc cagagcttcc tgttcgacgt gtccagcctt 1020
   aacgacgcag acgaggtggt gggtgccgag ctgcgcgtgc tgcgccgggg atctccagag 1080
   tcgggcccag gcagctggac ttctccgccg ttgctgctgc tgtccacgtg cccgggcgcc 1140
   gcccgagcgc cacgcctgct gtactcgcgg gcagctgagc ccctagtcgg tcagcgctgg 1200
   gaggcgttcg acgtggcgga cgccatgagg cgccaccgtc gtgaaccgcg ccccccccgc 1260
   gcgttctgcc tcttgctgcg cgcagtggca ggcccggtgc cgagcccgtt ggcactgcgg 1320
   cggctgggct tcggctggcc gggcggaggg ggctctgcgg cagaggagcg cgcggtgcta 1380
   gtcgtctcct cccgcacgca gaggaaagag agcttattcc gggagatccg cgcccaggcc 1440
   cgcgcgctcg gggccgctct ggcctcagag ccgctgcccg acccaggaac cggcaccgcg 1500
   tcgccaaggg cagtcattgg cggccgcaga cggaggagga cggcgttggc cgggacgcgg 1560
   acatcgcagg gcagcggcgg gggcgcgggc cggggccacg ggcgcagggg ccggagccgc 1620
   tgcagccgca agccgttgca cgtggacttc aaggagctcg gctgggacga ctggatcatc 1680
   gcgccgctgg actacgaggc gtaccactgc gagggccttt gcgacttccc tttgcgttcg 1740
   cacctcgagc ccaccaacca tgccatcatt cagacgctgc tcaactccat ggcaccagac 1800
   gcggcgccgg cctcctgctg tgtgccagcg cgcctcagcc ccatcagcat cctctacatc 1860
   gacgccgcca acaacgttgt ctacaagcaa tacgaggaca tggtggtgga ggcctgcggc 1920
   tgcaggtagc gcgagggccg gggagggggc agccacgcgg ccgaggatcc ccagctgatg 1980
   agcagcagcg ggcc 1994

## Description

### Technical Field

The invention generally relates to compositions comprising a crystal of BMP-7 for use in a method of implantation into the inter-articular space of a joint.

### Background

Bone morphogenetic proteins (BMPs) belong to the superfamily of transforming growth factor β (TGF-β), and control a diverse set of cellular and developmental processes, such as pattern formation and tissue specification as well as promoting wound healing and repair processes in adult tissues. BMPs were initially isolated by their ability to induce bone and cartilage formation. BMP signaling is inducible upon bone fracture and related tissue injury, leading to bone regeneration and repair.

To date, a reliable means for delivering a clinically effective dose of a BMP over a prolonged period of time, without repeated administration of the BMP, has heretofore eluded the skilled practitioner. In fact, sustained delivery of proteinaceous BAs generally remains an unanswered challenge. Moreover, despite progress in protein technologies and pharmaceutical chemistries, at least two problems continue to plague clinicians needing to provide sustained levels of key physiological factors to patients.

First, most therapeutic agents are administered orally. However, oral administration and other conventional drug delivery methods often are inappropriate for macromolecular drugs, as many of them are unstable in the blood stream and/or gastrointestinal tract, are toxic at high doses or have a narrow therapeutically effective concentration range (therapeutic window). This is further complicated in the case of chondral or osteochondral diseases and/or diseases or injuries of the joint since such tissues are poorly vascularized and not susceptible to treatment using some routine modes of systemic administration. Additionally, therapeutic proteins, for example, are typically administered by frequent injection because proteins generally have short *in vivo* half-lives and/or negligible oral bio-availability. This poses a substantial physical burden on the patient and creates significant administrative costs related to patient management. To provide greater efficacy, safety, patient convenience, and patient compliance, much effort has been spent attempting to develop and evaluate improved sustained-release formulations for protein and other macromolecular drugs. At the very least, a sustained release modality which permits sustained local release *via* a single administration would be desirable.

Second, formulations that obviate the need for the active ingredient to be prepared with a barrier, vehicle, or other inactive agents eliminate a great deal of the complexity inherent in manufacturing a dosage form. Other benefits of such comparatively simple dosage forms include lower manufacturing costs as well as the potential for higher active yields. Thus a modality that does not require carriers, vehicles, or other inactive agents would provide the skilled artisan with a preferable alternative means for administering biologically active agents systemically or locally.

Thus, there is a need for additional sustained delivery formulations suitable for administering biologically active agents, especially macromolecules such as BMPs and other proteinaceous macromolecular biologics or drugs.

Luginbuehl et al. (2004) European Journal of Pharmaceutics and Biopharmaceutics 58: 197-208 describe the crystallization of growth factors generally, and TGF-beta3 specifically, for sustained release purposes.

### Summary of the Invention

According to the present invention there is provided a composition comprising a crystal of BMP-7 for use in a method of implantation into the inter-articular space of a joint whereat said BMP-7 is released in a sustained-release manner to ameliorate an injury or disease of the joint.

Other aspects of the invention are as specified in claims 2-8.

The foregoing, and other features and advantages of the invention as well as the invention itself, will be more fully understood from the following figures, description, and claims.

### Brief Description of figures

Figure 1 comprises photographs at 1, 5, 22, and 96 hours (from left to right) of a BMP-7 crystal transferred into 50 mM acetic acid (pH 4) at room temperature.

Figure 2 comprises photographs at 1, 5, 22, and 96 hours (from left to right) of a BMP-7 crystal transferred into phosphate buffered saline (PBS) at room temperature.

Figure 3 comprises photographs at 1,5,22, and 96 hours (from left to right) of a BMP-7 crystal transferred into bovine synovial fluid at room temperature.

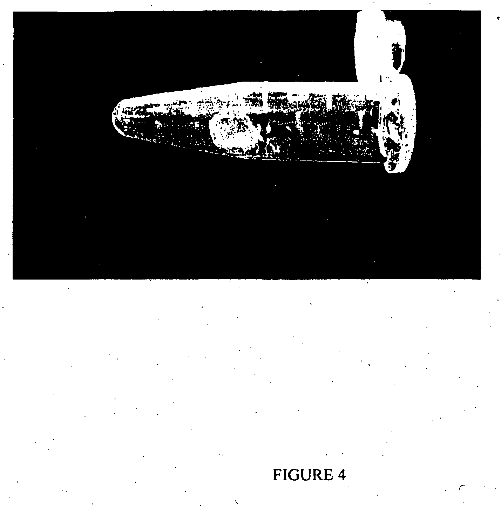

Figure 4 comprises a photograph of a high concentration protein gel of BMP-7 right after its production by centrifugal concentration in 50mM acetic acid.

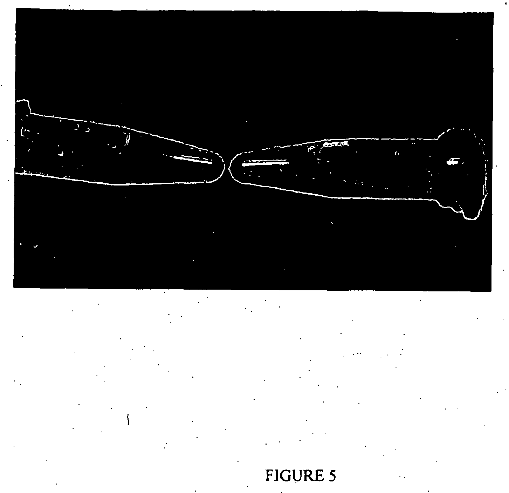

Figure 5 comprises a photograph of a high concentration protein gel of BMP-7 after 24 hours of rocking in 50mM acetic acid at 37 degrees Celsius.

### Detailed Description

The present invention is based on the discovery that BMP-7, can be formulated to provide a sustained release composition having ameliorative and restorative effects on injured, diseased or damaged cartilage without an associated inflammatory or irritative response at the site of intra-joint or intraminiscal administration.. BMP-7 belongs to the TGF-β superfamily. The TGF-β superfamily proteins are cytokines characterized by six-conserved cysteine residues). The human genome contains about 42 open reading frames encoding TGF-β superfamily proteins. The TGF-β superfamily proteins can at least be divided into the BMP subfamily and the TGF-β subfamily based on sequence similarity and the specific signaling pathways that they activate. The BMP subfamily includes, but is not limited to, BMP-2, BMP-3 (osteogenin), BMP-3b (GDF-10), BMP-4 (BMP-2b), BMP-5, BMP-6, BMP-7 (osteogenic protein-1 or OP-1), BMP-8 (OP-2), BMP-8B (OP-3), BMP-9 (GDF-2), BMP-10, BMP-11 (GDF-11), BMP-12 (GDF-7), BMP-13 (GDF-6, CDMP-2), BMP-15 (GDF-9), BMP-16, GDF-1, GDF-3, GDF-5 (CDMP-1, MP-52), and GDF-8 (myostatin). BMPs are also present in other animal species. Furthermore, there is allelic variation in BMP sequences among different members of the human population, and there is species variation among BMPs discovered and characterized to date. As used herein, "BMP subfamily," "BMPs," "BMP ligands" and grammatical equivalents thereof refer to the BMP subfamily members, unless specifically indicated otherwise.

The TGF-β subfamily includes, but is not limited to, TGFs (e.g., TGF-β1, TGF-β2, and TGF-β3), activins (e.g., activin A) and inhibins, macrophage inhibitory cytokine-1 (MIC-1), Mullerian inhibiting substance, anti-Mullerian hormone, and glial cell line derived neurotrophic factor (GDNF). As used herein, "TGF-β subfamily," "TGF-βs," "TGF-β ligands" and grammatical equivalents thereof refer to the TGF-β subfamily members, unless specifically indicated otherwise.

The TGF-β superfamily is in turn a subset of the cysteine knot Cytokine superfamily. Additional members of the cysteine knot cytokine superfamily include, but are not limited to, platelet derived growth factor (PDGF), vascular endothelial growth factor (VEGF), placenta growth factor (PIGF), noggin, neurotrophins (BDNF, NT3, NT4, and βNGF), gonadotropin, follitropin, lutropin, interleukin-17, and coagulogen.

Publications disclosing these sequences, as well as their chemical and physical properties, include: BMP-7 and OP-2 (U.S. Pat. No. 5,011,691; U.S. Pat. No. 5,266,683; Ozkaynak et al., EMBO J., 9, pp. 2085-2093 (1990); OP-3 (WO94/10203 (PCT US93/10520)), BMP-2, BMP-4, (WO88/00205; Wozney et al. Science, 242, pp. 1528-1534 (1988)), BMP-5 and BMP-6, (Celeste et al., PNAS, 87, 9843-9847 (1990)), Vgr-1 (Lyons et al., PNAS, 86, pp. 4554-4558 (1989)); DPP (Padgett et al. Nature, 325, pp. 81-84 (1987)); Vg-1 (Weeks, Cell, 51, pp. 861-867 (1987)); BMP-9 (WO95/33830 (PCT/US95/07084); BMP-10 (WO94/26893 (PCT/US94/05290); BMP-11 (WO94/26892 (PCT/US94/05288); BMP-12 (WO95/16035 (PCT/US94/14030); BMP-13 (WO95/16035 (PCT/US94/14030); GDF-1 (WO92/00382 (PCT/US91/04096) and Lee et al. PNAS, 88, pp. 4250-4254 (1991); GDF-8 (WO94/21681 (PCT/US94/03019); GDF-9 (WO94/15966 (PCT/US94/00685); GDF-10 (WO95/10539 (PCT/US94/11440); GDF-11 (WO96/01845 (PCT/US95/08543); BMP-15 (WO96/36710 (PCT/US96/06540); MP-121 (WO96/01316 (PCT/EP95/02552); GDF-5 (CDMP-1, MP52) (WO94/15949 (PCT/US94/00657) and WO96/14335 (PCT/US94/12814) and WO93/16099 (PCT/EP93/00350)); GDF-6 (CDMP-2, BMP13) (WO95/01801 (PCT/US94/07762) and WO96/14335 and WO95/10635 (PCT/US94/14030)); GDF-7 (CDMP-3, BMP12) (WO95/10802 (PCT/US94/07799) and WO95/10635 (PCT/US94/14030)) The above publications are incorporated herein by reference.

As used herein, "TGF-β superfamily member" or "TGF-β superfamily protein," means a protein known to those of ordinary skill in the art as a member of the Transforming Growth Factor- P (TGF-β) superfamily. Structurally, such proteins are homo or heterodimers expressed as large precursor polypeptide chains containing a hydrophobic signal sequence, an N-terminal pro region of several hundred amino acids, and a mature domain comprising a variable N-terminal region and a highly conserved C-terminal region containing approximately 100 amino acids with a characteristic cysteine motif having a conserved six or seven cysteine skeleton. These structurally-related proteins have been identified as being involved in a variety of developmental events.

The term "morphogenic protein" refers to a protein belonging to the TGF-ß superfamily of proteins which has true morphogenic activity. For instance, such a protein is capable of inducing progenitor cells to proliferate and/or to initiate a cascade of events in a differentiation pathway that leads to the formation of cartilage, bone, tendon, ligament, neural or other types of differentiated tissue, depending on local environmental cues. Thus, morphogenic proteins can behave differently in different surroundings.

The term "osteogenic protein (OP)" refers to a morphogenic protein that is also capable of inducing a progenitor cell to form cartilage and/or bone. The bone can be intramembranous bone or endochondral bone. Most osteogenic proteins are members of the BMP subfamily and are thus also BMPs. However, the converse can not be true. According to this invention, a BMP identified by DNA sequence homology or amino acid sequence identity must also have demonstrable osteogenic or chondrogenic activity in a functional bioassay to be an osteogenic protein. Appropriate bioassays are well known in the art; a particularly useful bioassay is the heterotopic bone formation assay (see, U.S. Pat. No. 5,011,691; U.S. Pat. No. 5,266,683, for example).

Structurally, BMPs are dimeric cysteine knot proteins. Each BMP monomer comprises multiple intramolecular disulfide bonds. An additional intermolecular disulfide bond mediates dimerization in most BMPs. BMPs may form homodimers. Some BMPs may form heterodimers. BMPs are expressed as pro-proteins comprising a long pro-domain, one or more cleavage sites, and a mature domain. The pro-domain is believed to aid in the correct folding and processing of BMPs. Furthermore, in some but not all BMPs, the pro-domain may noncovalently bind the mature domain and may act as an inhibitor (*e.g*., Thies et al. (2001) Growth Factors 18:251-259).

BMPs are naturally expressed as pro-proteins comprising a long pro-domain, one or more cleavage sites, and a mature domain. This pro-protein is then processed by the cellular machinery to yield a dimeric mature BMP molecule. The pro-domain is believed to aid in the correct folding and processing of BMPs. Furthermore, in some but not all BMPs, the pro-domain may noncovalently bind the mature domain and may act as a chaperone, as well as an inhibitor (e.g., Thies et. al. (2001) Growth Factors, 18:251-259).

BMP signal transduction is initiated when a BMP dimer binds two type I and two type II serine/threonine kinase receptors. Type I receptors include, but are not limited to, ALK-1, ALK-2 (also called ActRIa or ActRI), ALK-3 (also called BMPRIa), and ALK-6 (also called BMPRIb). Type II receptors include, but are not limited to, ActRIIa (also called ActRII), ActRIIb, and BMPRII. Human genome contains 12 members of the receptor serine/threonine kinase family, including 7 type I and 5 type II receptors, all of which are involved in TGF-β signaling (Manning et al., 2002, the disclosures of which are hereby incorporated by reference). Following BMP binding, the type II receptors phosphorylate the type I receptors, the type I receptors phosphorylate members of the Smad family of transcription factors, and the Smads translocate to the nucleus and activate the expression of a number of genes.

BMPs also interact with inhibitors, soluble receptors, and decoy receptors, including, but not limited to, BAMBI (BMP and activin membrane bound inhibitor), BMPER (BMP-binding endothelial cell precursor-derived regulator), Cerberus, cordin, cordin-like, Dan, Dante, follistatin, follistatin-related protein (FSRP), ectodin, gremlin, noggin, protein related to Dan and cerberus (PRDC), sclerostin, sclerostin-like, and uterine sensitization-associated gene-1 (USAG-1). Furthermore, BMPs may interact with coreceptors, for example BMP-2 and BMP-4 bind the co-receptor DRAGON (Samad et. al. (2005) J. Biol. Chen.), and extracellular matrix components such as heparin sulfate and heparin (Irie et al. (2003) Biochem. Biophys. Res. Commun. 308: 858-865).

As contemplated herein, the term "BMP" refers to a protein belonging to the BMP subfamily of the TGF-β superfamily of proteins defined on the basis of DNA homology and amino acid sequence identity. A protein belongs to the BMP subfamily when it has at least 50% amino acid sequence identity with a known BMP subfamily member within the conserved C-terminal cysteine-rich domain that characterizes the BMP subfamily. Members of the BMP subfamily can have less than 50% DNA or amino acid sequence identity overall. As used herein, the term "BMP" further refers to proteins which are amino acid sequence variants, domain-swapped variants, and truncations and active fragments of naturally occurring bone morphogenetic proteins, as well as heterodimeric proteins formed from two different monomeric BMP peptides, such as BMP-2/7; BMP-4/7: BMP-2/6; BMP-2/5; BMP-4/7; BMP-4/5; and BMP-4/6 heterodimers. Suitable BMP variants and heterodimers include those set forth in US 2006/0235204; WO 07/087053; WO 05/097825; WO 00/020607; WO 00/020591; WO 00/020449; WO 05/113585; WO 95/016034 and WO93/009229.

In the case of skeletal disorders, a number of factors can cause or contribute to cartilage degeneration in mammals, including trauma and inflammatory disease. Damage to cells resulting from the effects of inflammatory response has been implicated as the cause of reduced cartilage function or loss of cartilage function in diseases of the joints (e.g., rheumatoid arthritis (RA) and osteoarthritis (OA)). In addition, autoimmune diseases such as systemic lupus erythematosis (SLE) and scleroderma can also be characterized by a degradation of connective tissue. In the case of some cartilage degenerative diseases such as osteoarthritis (OA), the mechanisms that turn the normal aging of articular cartilage into the pathological OA process are currently unknown. Each of the foregoing diseases can be effectively treated according to the present invention.

In a preferred embodiment, the invention can be used to treat a disease or injury resulting in cartilage degradation or a cartilage defect. For example, the formulations can be applied to a cartilage defect site, such as a degenerative intervertebral disc, or other fibrocartilaginous tissue, including a tendon, a ligament or a meniscus. Such methods are set out in U.S. Patent No. 6,958,149. The formulations of the invention can also be used to treat a defect or degeneration of particular cartilage, as set forth in published PCT application WO 05/115438, such as the cartilage lining of a joint, such as a synovial joint, including a knee, an elbow, a hip, or a shoulder. In this embodiment, the formulation is preferably injected into the synovial space of the joint. In another embodiment, the formulations of the invention are used to treat an articular cartilage defect site, such as a chondral defect or an osteochondral defect, in a joint. Such articular cartilage defects can be the result of a disease process, such as osteoarthritis or rheumatoid arthritis, or due to injury of the joint. Formulation and Administration

TheBMP-7, of the present invention can be formulated for administration to a mammal, preferably a human, in need thereof as part of a pharmaceutical composition. The composition can be administered by means including, but not limited to, direct injection or infusion of the crystal by syringe. Additionally, the crystal may be introduced to the tissue by means including, but not limited to, direct surgical implantation, endoscopy, catheterization, or lavage. If applied during surgery, the composition may be flowed onto the tissue, sprayed onto the tissue, painted onto the tissue, or any other means within the skill in the art.

In a preferred embodiment, the composition is applied, administered, injected, implanted or used in a non-vascularized tissue site. As used herein, "non-vascularized" refers to a tissue or tissue site in which vascularization is minimal or absent. Such non-vascularized tissue sites include, but are not limited to, the joints, preferably the inter-articular space, preferably the meniscus.

The composition may be administered in or with an appropriate carrier or bulking agent including, but not limited to, a biocompatible oil such as sesame oil, hyaluronic acid, cyclodextrins, lactose, raffinose, mannitol, carboxy methyl cellulose, thermo or chemo-responsive gels, sucrose acetate isobutyrate. The skilled artisan would understand that the bulking agent or carrier most amenable to the practice of the present invention would facilitate the delivery of the condensed dosage forms of the composition wherein the dosage volumes include, but are not limited to, volumes of 20µl or less. Suspension or bulking media, either water- or oil-based, that are optimal for use with the microemulsions or emulsions as well as the bulking/suspension media optimal for the maintenance of the crystals can also be easily comprehended by the skilled artisan. In a particularly preferred embodiment of the present invention, a bulking agent can be used in conjunction with a composition of the present invention that is substantially insoluble at physiological pH, to increase the dissolution of the crystal such that the bulking agent acts classically as a barrier to release of the BMP-7. It is within the skill in the art to practice the aforementioned embodiments of the present invention, as well as any and all variants and modifications of the present invention that the skilled artisan would recognize provide sustained, effective post-dosing release of the BMP-7 depot *in vivo.*

Still further, the BMP-7 solid crystals, liquid crystals, of the present invention can be administered to the mammal in need thereof either alone or in combination with another substance known to have a beneficial effect on tissue morphogenesis. Examples of such substances (herein, cofactors) include without limitation substances that promote tissue repair and regeneration and/or inhibit inflammation. Examples of useful cofactors for stimulating bone tissue growth in osteoporotic individuals, for example, include but are not limited to, vitamin D₃, calcitonin, prostaglandins, parathyroid hormone, dexamethasone, estrogen and IGF-I or IGF-II. Useful cofactors for nerve tissue repair and regeneration can include, but are not limited to, nerve growth factors. Other useful cofactors include symptom-alleviating cofactors, including, but not limited to, antiseptics, antibiotics, antiviral and antifungal agents, analgesics and anesthetics.

As will be appreciated by those skilled in the art, the concentration of the compounds described in a therapeutic composition will vary depending upon a number of factors, including without limitation the dosage of the drug to be administered, the chemical characteristics (e.g., hydrophobicity) of the compounds employed, and the route of administration. The preferred dosage of drug to be administered also is likely to depend on variables including, but not limited to, the type and extent of a disease, tissue loss or defect, the overall health status of the particular patient, the relative biological efficacy of the compound selected, the formulation of the compound, the presence and types of excipients in the formulation, and the route of administration. The therapeutic molecules of the present invention may be provided to an individual where typical doses range from about 10 ng/kg to about 1 g/kg of body weight per day; with a preferred dose range being from about 0.1 mg/kg to 100 mg/kg of body weight, and with a more particularly preferred dosage range of 10-1000 µg/dose. In a particularly preferred embodiment, a dose of 10-1000 µg of a BMP-7 crystal, gel, or particulate suspension is administered to an individual afflicted with osteoarthritis. The skilled clinician would appreciate that the effective doses of the present invention can be modified in light of numerous factors including, but not limited to, the indication, the pathology of the disease, and the physical characteristics of the individual. It is also clearly within the skill in the art to vary, modify, or optimize doses in view of any or all of the aforementioned factors.

Pursuant to the parameters and conditions of the invention, the release of the BMP-7 can be controlled. In particular, the rate and extent of release of the BMP-7 from an implant, implantable article, device and the like according to the invention can be controlled by variation of the polymer type and molecular weight, use of a rate modifying agent, use of plasticizers and leachable agents and the concentrations and kinds of thermoplastic polymer and BMP-7.

Rate modifying agents, plasticizers and leachable agents can be included to manage the rate of release of BMP-7 and the pliability of a matrix in which it is optionally contained. The rate modifying agent can increase or retard the rate of release depending upon the nature of the rate modifying agent incorporated into a matrix. Known plasticizers as well as organic compounds that are suitable for secondary pseudobonding in polymer systems are acceptable as rate modifying agents and also as pliability modifiers and leaching agents. Generally these agents are esters of mono, di and tricarboxylic acids, diols and polyols, polyethers, non-ionic surfactants, fatty acids, fatty acid esters, oils such as vegetable oils, and the like. The concentrations of such agents within the matrix can range in amount up to 60 wt % relative to the total weight of the matrix, preferably up to 30 wt % and more preferably up to 15 wt %. Generally, these rate modifying agents, leaching agents, plasticizers and pliability modifiers and their application are described in U.S. Pat. No's. 5,702,716 and 5,447,725, the disclosures of which are incorporated herein by reference with the proviso that the polymers to be used are biocompatible and/or biodegradable. The skilled artisan would appreciate that the present invention comprises any and all agents within the art that can increase the solubilization rate of the BMP-7 or the degradation rate or erosion rate of any carrier for the BMP-7. Hence, other agents amenable to the practice of the present invention include, but are not limited to, co-localized pH modifying agents and tonicity modifiers. In a particularly preferred embodiment, the composition of the present invention comprises a co-localized pH modifying agent or tonicity modifier provided in a concentration or quantity that substantially increases the solubilization rate of the BMP-7. In another preferred embodiment, the composition of the present invention comprises a co-localized pH modifying agent or tonicity modifier provided in a concentration or quantity that substantially increases the degradation rate or erosion rate of the carrier. The skilled artisan would appreciate that the rate modifying agents, leaching agents, plasticizers, pliability modifiers, pH modifying agents, and tonicity modifiers of the present invention can be substituted, modified, varied in nature or concentration, and optimized in view of numerous factors, including, but not limited to, the desired release rate, the nature of the carrier (if any), the indication, the pathology of the disease, and the physical characteristics of the individual.

Controlled dissolution of the solid or liquid protein crystal, crystal formulation, or release of the constituent of any formulations can be controlled by numerous factors, including, but not limited to, the surface area of the crystal, particle, or gel; the size of said crystal, particle, or gel; the shape of said crystal, particle or gel; the concentration of any excipient component; the number and nature of any excipient components; the molecular weight of any excipient components; and any combinations of the aforementioned.

Organic solvent, water, or any other fluid may be removed from the crystal by any means including, but not limited to, drying with nitrogen, air or inert gases; vacuum oven drying; lyophilization; washing with a volatile organic solvent followed by evaporation; evaporation in a fume hood; passing a stream of gas over wet crystals, the gas being nitrogen, a Noble gas, carbon dioxide, air, or combinations thereof; or exchange into a biocompatible solvent or aqueous based system for storage and delivery.

Formulations of crystals according to the invention can include a combination of the crystal and one ore more ingredients or excipients, including sugars and biocompatible polymers. Examples of excipients are described in the Handbook of Pharmaceutical Excipients, published jointly by the American Pharmaceutical Association and the Pharmaceutical Society of Great Britain. For the purposes of this application, "formulations" include "crystal formulations." Furthermore, "formulations" include "protein crystal formulations," "protein gel formulations," and "protein suspension formulations."

As used herein "pharmaceutically effective amount" means an amount of a BMP-7 crystal, that is effective to treat a condition in a living organism to which it is administered over a period of time.

Excipients that may be employed in the making and use of the formulations and pharmaceutical compositions of the present invention include, but are not limited to; acidifying agents, such as, acetic acid, glacial acetic acid, citric acid, fumaric acid, hydrochloric acid, diluted hydrochloric acid, malic acid, nitric acid, phosphoric acid, diluted phosphoric acid, sulfuric acid, tartaric acid; alcohol denaturants, such as, denatonium benzoate, methyl isobutyl ketone, sucrose octacetate; alkalizing agents, such as, strong ammonia solution, ammonium carbonate, diethanolamine, diisopropanolamine, potassium hydroxide, sodium bicarbonate, sodium borate, sodium carbonate, sodium hydroxide, trolamine; antifoaming agents, such as, dimethicone, simethicone; antimicrobial preservatives, such as, benzalkonium chloride, benzalkonium chloride solution, benzelthonium chloride, benzoic acid, benzyl alcohol, butylparaben, cetylpyridinium chloride, chlorobutanol, chlorocresol, cresol, dehydroacetic acid, ethylparaben, methylparaben, methylparaben sodium, phenol, phenylethyl alcohol, phenylmercuric acetate, phenylmercuric nitrate, potassium benzoate, potassium sorbate, propylparaben, propylparaben sodium, sodium benzoate, sodium dehydroacetate, sodium propionate, sorbic acid, thimerosal, thymol; antioxidants, such as, ascorbic acid, acorbyl palmitate, butylated hydroxyanisole, butylated hydroxytoluene, hypophosphorous acid, monothioglycerol, propyl gallate, sodium formaldehyde sulfoxylate, sodium metabisulfite, sodium thiosulfate, sufur dioxide, tocopherol, tocopherols excipient; buffering agents, such as, acetic acid, ammonium carbonate, ammonium phosphate, boric acid, citric acid, lactic acid, phosphoric acid, potassium citrate, potassium metaphosphate, potassium phosphate monobasic, sodium acetate, sodium citrate, sodium lactate solution, dibasic sodium phosphate, monobasic sodium phosphate; chelating agents, such as, edetate disodium, ethylenediaminetetraacetic acid and salts, edetic acid; coating agents, such as, sodium carboxymethylcellulose, cellulose acetate, cellulose acetate phthalate, ethylcellulose, gelatin, pharmaceutical glaze, hydroxypropyl cellulose, hydroxypropyl methylcellulose, hydroxypropyl methylcellulose phthalate, methacrylic acid copolymer, methylcellulose, polyethylene glycol, polyvinyl acetate phthalate, shellac, sucrose, titanium dioxide, carnauba wax, microcystalline wax, zein; colors, such as, caramel, red, yellow, black or blends, ferric oxide; complexing agents, such as, ethylenediaminetetraacetic acid and salts (EDTA), edetic acid, gentisic acid ethanolmaide, oxyquinoline sulfate ; dessicants, such as, calcium chloride, calcium sulfate, silicon dioxide; emulsifying and/or solubilizing agents, such as, acacia, cholesterol, diethanolamine (adjunct), glyceryl monostearate, lanolin alcohols, lecithin, mono- and di-glycerides, monoethanolamine (adjunct), oleic acid (adjunct), oleyl alcohol (stabilizer), poloxamer, polyoxyethylene 50 stearate, polyoxyl 35 caster oil, polyoxyl 40 hydrogenated castor oil, polyoxyl 10 oleyl ether, polyoxyl 20 cetostearyl ether, polyoxyl 40 stearate, polysorbate 20, polysorbate 40, polysorbate 60, polysorbate 80, propylene glycol diacetate, propylene glycol monostearate, sodium lauryl sulfate, sodium stearate, sorbitan monolaurate, soritan monooleate, sorbitan monopalmitate, sorbitan monostearate, stearic acid, trolamine, emulsifying wax; filtering aids, such as, powdered cellulose, purified siliceous earth; glidants and/or anticaking agents, such as, calcium silicate, magnesium silicate, colloidal silicon dioxide, talc; humectants, such as, glycerin, hexylene glycol, propylene glycol, sorbitol; plasticizers, such as, castor oil, diacetylated monoglycerides, diethyl phthalate, glycerin, mono- and di-acetylated monoglycerides, polyethylene glycol, propylene glycol, triacetin, triethyl citrate; polymer membranes, such as, cellulose acetate; solvents, such as, acetone, acetic acid, alcohol, diluted alcohol, amylene hydrate, benzyl benzoate, butyl alcohol, carbon tetrachloride, chloroform, corn oil, cottonseed oil, ethyl acetate, glycerin, hexylene glycol, isopropyl alcohol, methyl alcohol, methylene chloride, methyl isobutyl ketone, mineral oil, peanut oil, polyethylene glycol, propylene carbonate, propylene glycol, sesame oil, water for injection, sterile water for injection, sterile water for irrigation, purified water; sorbents, such as, powdered cellulose, charcoal, purified siliceous earth, and carbon dioxide sorbents; stiffening agents, such as, hydrogenated castor oil, cetostearyl alcohol, cetyl alcohol, cetyl esters wax, hard fat, paraffin, polyethylene excipient, stearyl alcohol, emulsifying wax, white wax, yellow wax; suspending and/or viscosity-increasing agents, such as, acacia, agar, alginic acid, aluminum monostearate, bentonite, purified bentonite, magma bentonite, carbomer 934p, carboxymethylcellulose calcium, carboxymethylcellulose sodium, carboxymethycellulose sodium 12, carrageenan, microcrystalline and carboxymethylcellulose sodium cellulose, dextrin, gelatin, guar gum, hydroxyethyl cellulose, hydroxypropyl cellulose, hydroxypropyl methylcellulose, magnesium aluminum silicate, methylcellulose, pectin, polyethylene oxide, polyvinyl alcohol, povidone, propylene glycol alginate, silicon dioxide, colloidal silicon dioxide, sodium alginate, tragacanth, xanthan gum; and wetting and/or solubilizing agents, such as, benzalkonium chloride, benzethonium chloride, cetylpyridinium chloride, docusate sodium, nonoxynol 9, nonoxynol 10, octoxynol 9, poloxamer, polyoxyl 35 castor oil, polyoxyl 40, hydrogenated castor oil, polyoxyl 50 stearate, polyoxyl 10 oleyl ether, polyoxyl 20, cetostearyl ether, polyoxyl 40 stearate, polysorbate 20, polysorbate 40, polysorbate 60, polysorbate 80, sodium lauryl sulfate, sorbitan monolaureate, sorbitan monooleate, sorbitan monopalmitate, sorbitan monostearate, tyloxapol.

### Bioactive Co-agents

The present invention also contemplates "bioactive co-agents" that can be co-administered with the BMP-7 crystal of the present invention. These include, but are not limited to, anabolic agents, antacids, anti-asthmatic agents, anti-cholesterolemic and anti-lipid agents, anti-coagulants, anti-convulsants, anti-diarrheals, anti-emetics, anti-infective agents including, for example, antibacterial and antimicrobial agents, anti-inflammatory agents, anti-manic agents, antimetabolite agents, anti-nauseants, anti-neoplastic agents, anti- bone resorption agents, anti-obesity agents, anti-pyretic and analgesic agents, antispasmodic agents, anti-thrombotic agents, anti-tussive agents, anti-uricemic agents, anti-anginal agents, antihistamines, appetite suppressants, biologicals, cerebral dilators, coronary dilators, bronchodilators, cytotoxic agents, decongestants, diuretics, diagnostic agents, erythropoietic agents, expectorants, gastrointestinal sedatives, hyperglycemic agents, hypnotics, hypoglycemic agents, immunomodulating agents, ion exchange resins, laxatives, mineral supplements, mucolytic agents, neuromuscular drugs, peripheral vasodilators, psychotropics, sedatives, stimulants, thyroid and anti-thyroid agents, tissue growth agents, uterine relaxants, vitamins, or antigenic materials.

More particularly, the bioactive co-agents preferred for co-administration with the crystals, gels, or particulate suspensions of the present invention include, but are not limited to, androgen inhibitors, polysaccharides, growth factors, hormones, bisphosphonates, anti-angiogenesis factors, dextromethorphan, dextromethorphan hydrobromide, noscapine, carbetapentane citrate, chlophedianol hydrochloride, chlorpheniramine maleate, phenindamine tartrate, pyrilamine maleate, doxylamine succinate, phenyltoloxamine citrate, phenylephrine hydrochloride, phenylpropanolamine hydrochloride, pseudoephedrine hydrochloride, ephedrine, codeine phosphate, codeine sulfate morphine, mineral supplements, cholestryramine, N-acetylprocainamide, acetaminophen, aspirin, ibuprofen, phenyl propanolamine hydrochloride, caffeine, guaifenesin, aluminum hydroxide, magnesium hydroxide, peptides, polypeptides, proteins, amino acids, hormones, interferons, cytokines, and vaccines. Other representative bioactive co-agents that can be co-administered with the crystalline, gel, and particulate suspension compositions of the present invention include, but are not limited to, peptide drugs, protein drugs, desensitizing materials, antigens, anti-infective agents such as antibiotics, antimicrobial agents, antiviral, antibacterial, antiparasitic, antifungal substances and combination thereof, antiallergenics, androgenic steroids, decongestants, hypnotics, steroidal anti-inflammatory agents, anti-cholinergics, sympathomimetics, sedatives, miotics, psychic energizers, tranquilizers, vaccines, estrogens, progestational agents, humoral agents, prostaglandins, analgesics, antispasmodics, antimalarials, antihistamines, cardioactive agents, nonsteroidal anti-inflammatory agents, antiparkinsonian agents, antihypertensive agents, .beta.-adrenergic blocking agents, nutritional agents, and the benzophenanthridine alkaloids. The bioactive co-agent may further be a substance capable of acting as a stimulant, sedative, hypnotic, analgesic, anticonvulsant, and the like.

The bioactive co-agent may also be a substance, or metabolic precursor thereof, which is capable of promoting growth and survival of cells and tissues, or augmenting the activity of functioning cells, as for example, blood cells, neurons, muscle, bone marrow, bone cells and tissues, and the like. For example, bioactive co-agents that may be co-administered with the crystalline, gel, or particulate suspension compositions of the present invention may include without limitation a nerve growth promoting substance, as for example, a ganglioside, phosphatidylserine, a nerve growth factor, brain-derived neurotrophic factor. The bioactive co-agent may also be a growth factor for soft or fibrous connective tissue as, for example, a fibroblast growth factor, an epidermal growth factor, an endothelial cell growth factor, a platelet derived growth factor, an insulin-like growth factor, a periodontal ligament cell growth factor, to name but a few.

### Crystallinity

The crystallization of macromolecules, including proteins, can greatly aid in their storage, as well as their *in vivo* delivery. However, stability of these crystals can present numerous problems, since there are very few methods for preparing large quantities of macromolecule crystals that are stable outside of the mother liquor. In particular, protein crystals must be handled with greater care since they are extremely fragile and contain a good deal of solvent. One technique commonly employed allows for the separation of the crystal from the mother liquor and its insertion into a capillary tube with subsequent airtight scaling of the tube using, for instance, dental wax or silicone grease, along with a small amount of the mother liquor to maintain the crystal's hydration. (McPherson, A., Preparation and Analysis of Protein Crystals, Robert E. Krieger Publishing, Malabar, p. 214 (1989)). Macromolecular crystals can also be maintained at cryogenic temperatures using methods well known in the art. Preparation of the crystal with subsequent rapid cooling can prevent the formation of ice lattices within the aqueous medium. In lieu of the ice that would normally form, a rigid glass forms instead, encasing the crystal without damaging it. The resulting crystals are stored at 100K to prevent disintegration of the crystal. (Rodgers, D. W., in Methods in Enzymology (Eds., Carter, C. W. and Sweet, R. M.) Academic Press, v.276, p. 183 (1997)). Although this technique allows storage of crystals outside of the mother liquor, it requires maintenance of the crystal at temperatures at or below 100K.

Dried crystals can also be prepared by lyophilization, a technique that requires rapid cooling of the material. This limits the application of the technique to products that are stable under such frozen conditions. The technique requires that the aqueous solution is frozen first at a temperature of between -40 and -50 degrees Celsius. The resulting ice is then removed under vacuum, since ice formulation can potentially destroy the protein crystal lattice.

Optimally, crystalline macromolecules should be stable at ambient temperatures for convenient storage. Crystalline macromolecules, particularly crystalline proteins, are particularly advantageous for use as therapeutics and vaccines. The present invention provides formulations and compositions of crystalline BMP-7, that are solid particles or dispersed in a non-aqueous solvent. In an embodiment of the present invention, the BMP-7 compositions of the present invention comprise, in place of the mother liquor, a non-aqueous solvent. In another embodiment of the present invention, a slurry of the crystalline BMP-7 can be rendered solid by spinning out the first solvent and washing the remaining crystalline BA solid using a second organic solvent to remove water with subsequent evaporation of the non-aqueous solvent.

To optimize the preparation and maintenance of protein crystals, it is possible to leave the crystals in the mother liquor during the course of the protein crystal production process, potentially encapsulated in polymeric carriers. Polymer processing conditions are compatible with the many of the compounds used in protein crystallization including, but not limited to, salts, PEG, and organic solvents. The skilled artisan would also appreciate that crystal dissolution within the mother liquor can be controlled by conditions including, but not limited to, pH; temperature; presence of metal ions, such as Zn, Cu and Ca; and the concentration of precipitants. The skilled artisan would also recognize that, by varying these conditions, one can slow down the dissolution of crystals for several hours. The skilled artisan would further appreciate that the process of microparticulate formation is very fast and normally takes seconds to minutes to complete. Furthermore, filtration can be used to remove the mother liquor, leaving a crystalline paste that can be dried by air, under vacuum, washing with miscible organic solvents, and/or by lyophilization, leaving dried crystals. The skilled artisan would also appreciate that crystals, including protein crystals, can be chemically crosslinked to greatly reduce, or eliminate altogether, the propensity to dissolve in aqueous, or even non-aqueous, media. It is also within the art to manipulate or control the crystal size or shape during the crystallization process, resulting in a range of crystal morphologies with differing dissolution kinetics and, therefore, differing sustained release profiles compared to amorphous proteins.

In another embodiment of this invention, an excipient is dissolved in a solution other than the mother liquor, and the BMP-7 crystals are removed from the mother liquor and suspended in the excipient solution.

The skilled artisan would also appreciate that macromolecules, such as BMP-7, are easier to crystallize, and have more stable resulting crystals and gels, if the macromolecules have low solubility and have tertiary and/or quaternary structures that are relatively conformationally immobile. In particular, proteins that have strong interactions, including, but not limited to, covalent bonds between tertiary structures or between polypeptides in a multimer, for instance, have fewer conformational degrees of freedom than proteins lacking such interactions. The decreased conformational mobility makes the proteins more amenable to the local ordering that may aid crystallization and gel-formation. Furthermore, proteins with low solubility also tend to aggregate, their hydrophobic surfaces forming, for instance, extensive Van der Waals contacts that encourage local ordering of the proteins which in turn may aid in crystallization and gel-formation. The skilled artisan would appreciate that the proteins of the TGF-β superfamily and especially the BMPs are, relative to other proteins, conformationally immobile and substantially physiologically insoluble, and are therefore particularly amenable to the making and use of the crystals, gels, and particulate suspensions of the present invention. The skilled artisan would appreciate that varying degrees of solubility and conformational immobility can alter the nature and morphology of crystals and it is also within the art for the routineer to modify and vary the conditions under which such proteins optimally crystallize.

The possible advantage of the crystalline form as opposed to a pre-precipitated form is the reduced surface area to volume ratio which can increase sustained release levels. The crystalline form, with its reduced surface area to volume ratio, is also likely less irritating to tissues at the site of administration since the lower surface area per given dose mitigates or reduces the local irritation from precipitation. In a preferred embodiment, the BMP-7 crystals can be administered using a syringe with a gauge between 12 and 30. In a still more particularly preferred embodiment, the BMP-7 crystals can be administered using a syringe with a gauge between 16 and 26. The skilled artisan would appreciate that the manipulation of the surface area/volume ratio of the BMP-7 crystals of the present invention can modify the dissolution/release rate according to her desires with such manipulation well within the skill in the art.

The present invention also envisions the practice of all means known and commonly used in the art for crystallizing proteins including, but not limited to, concentration-through-evaporation, sublimation, diffusion gradient techniques, and batch techniques.

### Pharmaceutical Compositions

The pharmaceutical compositions of the present invention can comprise one or more other excipients or agents disclosed herein above including, but not limited to, release modifying agents, plasticizers, carriers, pliability modifiers, tonicity modifiers, or co-localized pH modifying agents. The skilled artisan would appreciate that the pharmaceutical compositions of the present invention can be modified or varied to optimize treatment of an individual in view of numerous factors including, but not limited to, the indication, the pathology of the disease, and the physical characteristics of the individual.

### Kits

The composition of the present invention may be presented in the form of kits useful for the treatment of disease, particularly joints impacted by disease, especially osteoarthritis and osteochondral disease. In a preferred embodiment, the kits of the present invention comprise one or more additional biologically active agents.. The kits of the present invention can also comprise one or more other excipients or agents disclosed herein above including, but not limited to, release modifying agents, plasticizers, carriers, pliability modifiers, tonicity modifiers, co-localized pH modifying agents, or pharmaceutically acceptable solvents and vehicles. The skilled artisan would appreciate that the kits of the present invention can be modified or varied to optimize treatment of an individual in view of numerous factors including, but not limited to, the indication, the pathology of the disease, and the physical characteristics of the individual.

### Examples

### 1. Crystals and Protein Kinetics Modeling

BMP-7 crystals were grown by vapor diffusion methods in a sitting drop tray at 19 degrees C. One well contained multiple crystals at approximately 0.1 mm size which were produced using 7.7 mg/mL of BMP-7, with a well solution of 16% 2-methyl-2,4,-pentandiol (MPD) and 135 mM sodium citrate (pH 4.8).

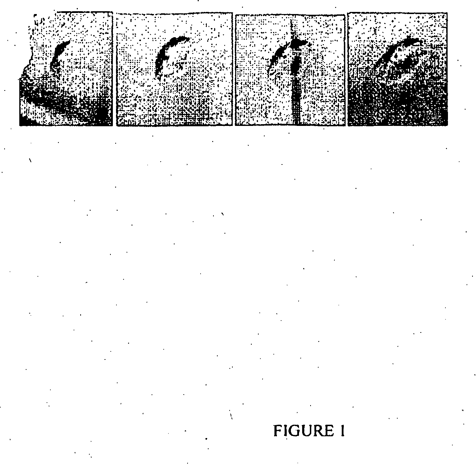

In a sitting drop crystallization tray, 35 microliters of test solution was placed into the post. A crystal was manually transferred using a loop into teach of three solutions: 50 mM acetic acid, phosphate buffered saline (PBS), and bovine synovial fluid. The crystals were observed by a stereo microscope and photographed at 1, 5, 22, and 96 hours with storage under ambient room temperature (approximately 19 degrees C) in each of the three solutions (Figs. 1-3).

The crystal that was transferred into 50 mM acetic acid was the least stable (Fig. 1). The edges were observed to have slightly dissolved within the first hour of transfer. Further degradation of the crystal was observed with prolonged exposure.

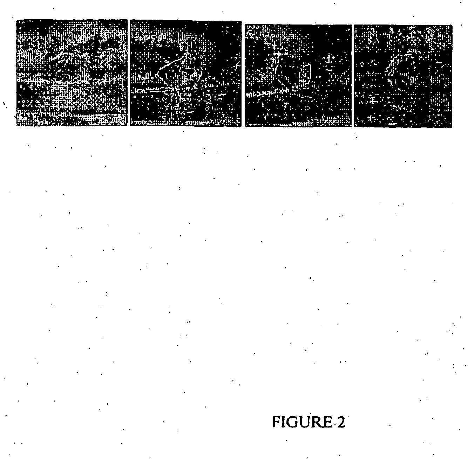

When the crystal was transferred into PBS, a few cracks were produced in the crystal during the initial equilibration (Fig. 2). Prolonged storage in PBS did not result in significant observable changes in the crystal.

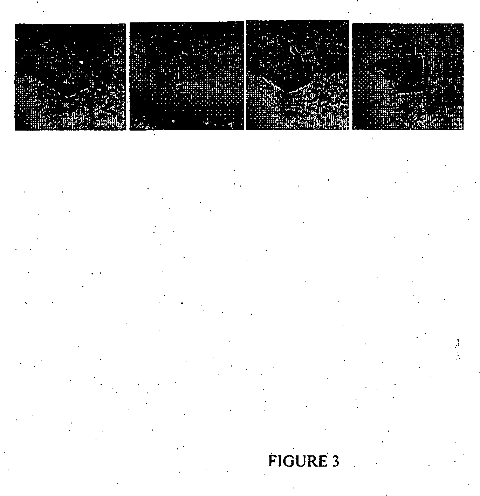

When a crystal was transferred into bovine synovial fluid, some internal cracking was observed (Fig. 3). Further equilibration in the synovial fluid did not appear to alter the edges of the crystal.

These results indicate such a crystal would provide a sustained release depot in the knee to stimulate cartilage repair, for instance. The size of the crystal (greater than the MW cut off of the synovial membrane) helps retain the material in the knee, and provides prolonged delivery time for the protein due to slow dissolution.

The release profile of the BMP crystals may be manipulated to give desired release kinetics. For instance, by injecting a pre-precipitated dose like BMP-7 crystals or a lyophilized BMP-7 protein suspended in saline higher sustained release levels may be reached and a lower Cₘₐₓ level may be achieved. Furthermore, the release rate may be regulated by local injection of solubilized protein, i.e., suspended in saline, thus shifting the release equilibrium. This can take the form of either co-administration with the crystal or protein gel, or can take place as a secondary administration after the initial administration of the crystal or the protein gel.

## Claims

1. A composition comprising a crystal of BMP-7 for use in a method of implantation into the inter-articular space of a joint whereat said BMP-7 is released in a sustained-release manner to ameliorate an injury or disease of the joint.

2. The composition for use according to claim 1 wherein the crystal is formed *ex vivo.*

3. The composition for use according to claim 1 or claim 2 wherein the composition is in an amount effective to ameliorate: (a) skeletal tissue injury or disease selected from metabolic bone disease, osteoarthritis, osteochondral disease, rheumatoid arthritis, osteoporosis and Paget's disease; (b) non-mineralized skeletal tissue injury or disease selected from osteoarthritis, osteochondral disease, chondral disease, rheumatoid arthritis, trauma-induced and inflammation-induced cartilage degeneration, age-related cartilage degeneration, articular cartilage injuries and diseases, full thickness cartilage, defects, superficial cartilage defects, sequelae of systemic lupus erythematosis, sequelae of scleroderma, periodontal tissue regeneration, hierniation and rupture of intervertebral discs, degenerative diseases of the intervertebral disc, osteocondrosis, and injuries and diseases of ligament, tendon, synovial capsule, synovial membrane and meniscal tissues; (c) tissue injury selected from trauma-induced and inflammation-induced cartilage degeneration, articular cartilage injuries, full thickness cartilage defects, superficial cartilage defects, hierniation and rupture of intervertebral discs, degeneration of intervertebral discs due to an injury(s), and injuries of ligament, tendon, synovial capsule, synovial membrane and meniscal tissues.

4. The composition for use according to any one of the preceding claims wherein the biologic agent is released in a sustained release manner for at least about 2-7 days.

5. The composition for use according to any one of the preceding claims wherein said effective amount for treatment of osteoarthritis is about 10 to about 1000 micrograms.

6. The composition for use according to any one of the preceding claims further comprising a pharmaceutically-acceptable vehicle.

7. The composition for use according to any one of the preceding claims further comprising a release modifying agent.

8. The composition for use according to any one of the preceding claims further comprising a bulking agent.

## Patentansprüche

1. Zusammensetzung, welche einen BMP-7-Kristall umfasst, zur Verwendung in einem Verfahren zur Implantierung in den inter-artikulären Raum eines Gelenks, worin das BMP-7 in verzögerter Art und Weise freigesetzt wird, um eine Verletzung oder Erkrankung des Gelenks zu bessern.

2. Zusammensetzung zur Verwendung nach Anspruch 1, worin der Kristall ex vivo erzeugt wird.

3. Zusammensetzung zur Verwendung nach Anspruch 1 oder Anspruch 2, worin die Zusammensetzung in einer wirksamen Menge vorhanden ist, um zu bessern:
(a) eine Verletzung oder Erkrankung des Skelettgewebes ausgewählt unter metabolischer Knochenerkrankung, Osteoarthritis, osteochondraler Erkrankung, rheumatoider Arthritis, Osteoporose und Paget's Erkrankung,
(b) eine Verletzung oder Erkrankung von nicht mineralisiertem Skelettgewebe ausgewählt unter Osteoarthritis, osteochondraler Erkrankung, chondraler Erkrankung, rheumatoider Arthritis, Trauma-induzierter und Entzündungs-induzierter Knorpeldegenerierung, altersbedingter Knorpeldegenerierung, artikulärer Knorpel-Verletzungen und -Erkrankungen, den ganzen Knorpel betreffenden Defekten, Knorpeloberflächen-Defekten, Spätschäden von systemischem Lupus Erythematosus, Folgeschäden von Skleroderma, periodontaler Gewebe-Regenerierung, Hierniation und Bruch intervertebraler Scheiben, degenerativer Erkankungen von intervertebralen Scheiben, Osteokondrosis, und Verletzungen und Erkrankungen des Ligaments, der Sehnen, der synovialen Kapsel, der synovialen Membran und des Meniskus-Gewebes,
(c) Gewebe-Verletzungen ausgewählt unter Trauma-induzierter und Entzündungs-induzierter Knorpeldegeneration, artikulärer Knorpelverletzungen, den ganzen Knorpel betreffenden Defekten, Knorpeloberflächen-Defekten, Hierniation und Bruch intervertebraler Scheiben, Degeneration intervetrebraler Scheiben aufgrund von Verletzung(en), und Verletzungen des Ligaments, der Sehnen, der synovialen Kapsel, der synovialen Membran und des Meniskus-Gewebes.

4. Zusammensetzung zur Verwendung nach einem der vorhergehenden Ansprüche, worin das biologische Mittel in einer verzögerten Art und Weise für mindestens 7 Tage freigesetzt wird.

5. Zusammensetzung zur Verwendung nach einem der vorhergehenden Ansprüche, worin die wirksame Menge für die Behandlung von Osteoarthritis etwa 10 bis etwa 1000 µg beträgt.

6. Zusammensetzung zur Verwendung nach einem der vorhergehenden Ansprüche, welche weiter einen pharmazeutisch annehmbaren Träger umfasst.

7. Zusammensetzung zur Verwendung nach einem der vorhergehenden Ansprüche, welche weiter ein Freisetzungs-modifizierendes Mittel umfasst.

8. Zusammensetzung zur Verwendung nach einem der vorhergehenden Ansprüche, welche weiter ein Quellmittel umfasst.

## Revendications

1. Composition comprenant un cristal de BMP-7 pour une utilisation dans un procédé d'implantation dans l'espace interarticulaire d'une articulation au niveau de laquelle ledit BMP-7 est libéré de manière prolongée pour améliorer une lésion ou une maladie de l'articulation.

2. Composition pour une utilisation selon la revendication 1, dans laquelle le cristal est formé ex vivo.

3. Composition pour une utilisation selon la revendication 1 ou la revendication 2, dans laquelle la composition est en une quantité efficace pour améliorer : (a) une lésion ou maladie d'un tissu squelettique choisie parmi une maladie osseuse métabolique, l'arthrose, une maladie ostéochondrale, la polyarthrite rhumatoïde, l'ostéoporose et la maladie de Paget ; (b) une lésion ou maladie d'un tissu squelettique non minéralisé choisie parmi l'arthrose, une maladie ostéochondrale, une maladie chondrale, la polyarthrite rhumatoïde, une dégénérescence du cartilage induite par un traumatisme et induite par une inflammation, la dégénérescence du cartilage liée à l'âge, les lésions et maladies du cartilage articulaire, les défauts du cartilage sur toute l'épaisseur, les défauts superficiels du cartilage, les séquelles du lupus érythémateux disséminé, les séquelles d'une sclérodermie, la régénération d'un tissu périodontale, une hernie et une rupture des disques intervertébraux, les maladies dégénératives des disques intervertébraux, l'ostéochondrose, et les lésions et maladies des ligaments, des tendons, de la capsule synoviale, de la membrane synoviale et des tissus méniscaux ; (c) une lésion d'un tissu choisie parmi une dégénérescence du cartilage induite par un traumatisme et induite par une inflammation, les lésions du cartilage articulaire, les défauts du cartilage sur toute l'épaisseur, les défauts superficiels du cartilage, une hernie et une rupture des disques intervertébraux, la dégénérescence des disques intervertébraux due à une ou plusieurs lésions, et les lésions des ligaments, des tendons, de la capsule synoviale, de la membrane synoviale et des tissus méniscaux.

4. Composition pour une utilisation selon l'une quelconque des revendications précédentes, dans laquelle l'agent biologique est libéré d'une manière prolongée pendant au moins environ 2 à 7 jours.

5. Composition pour une utilisation selon l'une quelconque des revendications précédentes, dans laquelle ladite quantité efficace pour le traitement de l'arthrose est d'environ 10 microgrammes à environ 1000 microgrammes.

6. Composition pour une utilisation selon l'une quelconque des revendications précédentes, comprenant en outre un véhicule pharmaceutiquement acceptable.

7. Composition pour une utilisation selon l'une quelconque des revendications précédentes, comprenant en outre un agent modifiant la libération.

8. Composition pour une utilisation selon l'une quelconque des revendications précédentes, comprenant en outre un diluant.
